Europäisches Patentamt

European Patent Office

Office européen des brevets

(19)

(11) **EP 1 171 627 B1**

(12) **EUROPEAN PATENT SPECIFICATION**

(45) Date of publication and mention
of the grant of the patent:
**27.10.2004 Bulletin 2004/44**

(51) Int Cl.⁷: **C12Q 1/00**

(86) International application number:
**PCT/GB2000/000663**

(21) Application number: **00906468.4**

(22) Date of filing: **25.02.2000**

(87) International publication number:
**WO 2000/050630 (31.08.2000 Gazette 2000/35)**

(54) **ASSAY FOR MEASURING DIFFERENT ENZYME ACTIVITIES SIMULTANEOUSLY**

TEST ZUR GLEICHZEITIGEN MESSUNG VON VERSCHIEDENEN ENZYMATISCHEN
AKTIVITÄTEN

DOSAGE PERMETTANT UNE MESURE SIMULTANEE DE L'ACTIVITE DE DIFFERENTES
ENZYMES

(84) Designated Contracting States:
**AT BE CH CY DE DK ES FI FR GB GR IE IT LI LU
MC NL PT SE**

(30) Priority: **25.02.1999 GB 9904401
25.02.1999 GB 9904392
25.02.1999 GB 9904393
25.02.1999 GB 9904398
25.02.1999 GB 9904395
25.02.1999 GB 9904407
13.01.2000 GB 0000771**

(43) Date of publication of application:
**16.01.2002 Bulletin 2002/03**

(73) Proprietor: **Cyclacel Limited
London SW1Y 4RB (GB)**

(72) Inventors:
• **COLYER, John
Bardsey LS17 9AN (GB)**
• **CRAIG, Roger KingdoN
Smallwood, Cheshire CW11 2XB (GB)**

(74) Representative: **Khoo, Chong-Yee
D Young & Co,
21 New Fetter Lane
London EC4A 1DA (GB)**

(56) References cited:
EP-A- 0 392 808          WO-A-92/00388
WO-A-96/12820          WO-A-96/20211

• W HE ET AL: "Grb10 Interacts Differentially with
the Insulin Receptor, Insulin-like Growth Factor
I Receptor, and Epidermal Growth Factor
Receptor via the Grb10 Src Homology (SH2)
Domain and a Second Novel Domain Located
between the Pleckstrin Homology and SH2
Domains" JOURNAL OF BIOLOGICAL
CHEMISTRY,US,AMERICAN SOCIETY OF
BIOLOGICAL CHEMISTS, BALTIMORE, MD, vol.
273, no. 12, 20 March 1998 (1998-03-20), pages
6860-6867, XP002124256 ISSN: 0021-9258
• RIEDEL H ET AL: "Cytoplasmic domains
determine signal specificity, cellular routing
characteristics and influence ligand binding of
epidermal growth factor and insulin receptors"
EMBO JOURNAL,GB,OXFORD UNIVERSITY
PRESS, SURREY, vol. 8, no. 10, 1989, pages
2943-2954, XP002130397 ISSN: 0261-4189
• CRAPARO A ET AL: "14-3-3 (EPSILON)
INTERACTS WITH THE INSULIN-LIKE GROWTH
FACTOR I RECEPTOR AND INSULIN RECEPTOR
SUBSTRATE I IN A PHOSPHOSERINE-
DEPENDENT MANNER" JOURNAL OF
BIOLOGICAL CHEMISTRY,US,AMERICAN
SOCIETY OF BIOLOGICAL CHEMISTS,
BALTIMORE, MD, April 1997 (1997-04), pages
11663-11669, XP002915059 ISSN: 0021-9258
• LAMOTHE B ET AL: "Reexamining interaction of
the SH2 domains of SYP and GAP with insulin
and IGF-1 receptors in the two-hybrid system"
GENE: AN INTERNATIONAL JOURNAL ON
GENES AND GENOMES,GB,ELSEVIER
SCIENCE PUBLISHERS, BARKING, vol. 182, no.
1-2, 5 December 1996 (1996-12-05), pages 77-80,
XP004071933 ISSN: 0378-1119

**Description**

The present invention relates to methods for simultaneously measuring the activity of two or more enzymes in the same system, typically in real time.

Enzymes play an important role in most if not all biological processes investigated to date. Although a variety of techniques exist for measuring enzyme activity, these techniques are suitable only for measuring the activity of one enzyme at a time. However, as studies of cellular pathways have progressed, it has become apparent that there is a complex interplay between the various components in those pathways. Accordingly, an assay system for measuring more than one enzyme activity at a time, particularly in complex systems such as whole cell assays or *in vivo* would be advantageous since it would be possible to measure how perturbation of a system affects concurrently the various enzyme activities within that system.

EP 0 392 808 A describes a method for determining the activity of an enzyme in a sample. A substrate labelled with a bioluminescent protein is exposed to a sample suspected of containing the enzyme to produce a product. A receptor which is capable of binding with either the labelled product or the labelled substrate (but not to both) is combined with the reaction medium to form a complex, and the presence or amount of detectable label is determined to establish the enzyme activity. Apart from bioluminescent labels, other types of labels are said to be not suitable for use in the method described in EP 0 392 808 A.

WO 96/12820 describes a method for identifying a compound which inhibits an interaction between a *CDC25* phophatase and a *Raf* kinase. The method involves an "interaction trap system" comprising two fusion proteins, one of which comprises a *Raf* protein portion, and the other comprising a *CDC25* protein portion. The *Raf* protein portion binds to the *CDC25* protein portion, and the system is exposed to a putative inhibitor. A decrease in the interaction of the fusion proteins in the presence of the candidate agent (compared to in the absence of it) indicates that the test agent is an inhibitor of the interaction.

We have now shown, using labelled reporter polypeptides whose interaction is modulated by post-translational modification of one or both or the polypeptides, that it is possible within the same reaction vessel to measure simultaneously the activity of two different enzymes.

Accordingly, the present invention provides a method of measuring simultaneously the activity of a first enzyme and a second enzyme in the same system which method comprises

(a) contacting a first binding domain and a first binding partner thereof with said first enzyme and contacting a second binding domain and a second binding partner thereof with said second enzyme; wherein

(i) the first binding domain and/or binding partner comprise a site subject to post-translational modification by the first enzyme;
(ii) modification of the site by the first enzyme affects the interaction between the first binding domain and first binding partner;
(iii) the second binding domain and/or binding partner comprise a site subject to post-translational modification by the second enzyme; and
(iv) modification of the site by the second enzyme affects the interaction between the second binding domain and second binding partner; and

(b) simultaneously measuring the interaction between the first binding domain and the first binding partner and measuring the interaction between the second binding domain and the second binding partner.

Preferably the first binding domain and first binding partner each comprise a detectable label such that when the first binding domain and first binding partner interact, a first detectable physical characteristic of one or both of the labels is altered; and
the second binding domain and second binding partner each comprise a detectable label such that when the second binding domain and second binding partner interact, a second detectable physical characteristic of one or both of the labels is altered; and
measurement of the interaction between the first binding domain and the first binding partner is performed by measuring changes in said first physical characteristic and measurement of the interaction between the second binding domain and the second binding partner is performed by measuring changes in said second physical characteristic, wherein the first physical characteristic is distinguishable from the second physical characteristic.

Preferably said physical characteristic is light emission or absorption. More preferably said physical characteristic is the emission of fluorescent light. Preferably the detectable label is a fluorophore such as a fluorescent protein or fluorescent chemical.

Preferably said first enzyme and/or said second enzyme is selected from a carbohydrate transferase, a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyl transferase, an NAD:Arginine ADP ribosyltransferase, a protease, a protein kinase and a protein phosphatase.

The first and second enzyme may carry out the same type of chemical modification (i.e., both are protein kinases for example) or different (e.g., the first is a kinase and the second is a protease).

In a preferred embodiment, the method further comprises the step, prior to, during or after measurement of the first and second physical characteristic, of contacting said first enzyme and/or second enzyme with a compound which modulates the activity of said first and/or second enzyme.

The present invention further provides a method of identifying a compound capable of modulating the activity of a first enzyme and/or second enzyme which method comprises

(i) contacting said first and/or second enzyme with a candidate substance
(ii) measuring the activity of the first and/or second enzyme by the method of Claim 1; and
(iii) determining whether the activity of the first and/or second enzyme is affected.

A compound capable of modulating the activity of a first and/or second enzyme which compound is identified by the above method may be used in therapy.

In a further embodiment, the present invention provides a set of two or more polypeptide pairs, each pair comprising:

(a) a binding domain and a binding partner thereof comprising

(i) a site subject to post-translational modification by an enzyme wherein modification of the site by the enzyme affects the interaction between the binding domain and the binding partner; and
(ii) a detectable label on the binding domain and binding partner such that when the binding domain and the binding partner interact, a detectable physical characteristic of one or both of the labels is altered;

wherein for each pair; the enzyme which affects the interaction between the binding domain and the binding partner is different and the detectable physical characteristic can be distinguished from that of the other pairs.

Also provided is the use of said set of polypeptide pairs in a method for measuring simultaneously the activity of two or more enzymes.

We also describe a kit comprising a set of polypeptide pairs as defined above. Also described are a set of nucleic acid sequences encoding the set of polypeptide pairs, a host cell comprising the set of nucleic acid sequences and a transgenic organism comprising the set of nucleic acid sequences.

Definitions

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook *et al*., Molecular Cloning: A Laboratory Manual, 2d ed. (1989) Cold Spring Harbor Laboratory Press, Cold Spring Harbor, N.Y. and Ausubel *et al*., Short Protocols in Molecular Biology (1999) 4th Ed, John Wiley & Sons, Inc.), chemical methods, pharmaceutical formulations and delivery and treatment of patients.

As used herein, the term "protein" includes single-chain polypeptide molecules as well as multiple-polypeptide complexes where individual constituent polypeptides are linked by covalent or non-covalent means. As used herein, the terms "polypeptide" and "peptide" refer to a polymer in which the monomers are amino acids and are joined together through peptide or disulfide bonds. The terms subunit and domain may also refer to polypeptides and peptides having biological function. A peptide useful in the invention will at least have a binding capability, i.e., with respect to binding as or to a binding partner, and also may have another biological function that is a biological function of a protein or domain from which the peptide sequence is derived. "Fragment thereof" typically refers to a selected region of the polypeptide that is of interest in a binding assay and for which a binding partner is known or determinable. "Fragment thereof' thus refers to an amino acid sequence that is a portion of a full-length polypeptide, between about 8 and about 1000 amino acids in length, preferably about 8 to about 300, more preferably about 8 to about 200 amino acids, and even more preferably about 10 to about 50 or 100 amino acids in length. "Peptide" refers to a short amino acid sequence that is 10 to 40 amino acids long, preferably 10 to 35 amino acids.

"Naturally-occurring" as used herein, as applied to a polypeptide or polynucleotide, refers to the fact that the polypeptide or polynucleotide can be found in nature. One such example is a polypeptide or polynucleotide sequence that is present in an organism (including a virus) that can be isolated from a source in nature.

As used herein, the terms "protein", "subunit" and "domain" refer to a linear sequence of amino acids which exhibits biological function. This linear sequence includes full-length amino acid sequences (e.g. those encoded by a full-length gene or polynucleotide), or a portion or fragment thereof, provided that portion or fragment maintains the biological function. The terms "subunit" and "domain" also may refer to polypeptides and peptides having biological function. A peptide useful in the invention will at least have a binding capability, i.e., with respect to binding as or to a binding partner, and also may have another biological function that is a biological function of a protein or domain from which the peptide sequence is derived.

"Polynucleotide" refers to a polymeric form of nucleotides of at least 10 bases in length and up to 1,000 bases or even more, either ribonucleotides or deoxyribonucleotides or a modified form of either type of nucleotide. The term includes single and double stranded forms of DNA.

A "system" refers to the medium in which the enzymes are present. For example the system may be a cell such that the enzymes, whose activity is to be simultaneously measured using the assays of the invention, are present in the same cell or it may be a reaction vessel such that the enzymes are present in the same reaction vessel, such as a tube or microtitre plate well. The situation where the enzymes are present in separate reaction vessels or cells and their activity measured separately, even if simultaneously, is specifically excluded from the definition of "in a system" or "in the same system".

A. Binding domains and binding partners

Reporter molecules may be used in methods for measuring the activity of protein modifying enzymes by measuring the interactions between the reporter molecules, said interactions being modulated by the activity of the protein modifying enzymes. These reporter molecules are typically naturally-occurring polypeptides which include natural binding domains, natural binding sequences and natural binding polypeptides, each as defined above, which are used in assays of the invention in combination with polypeptide binding partners, also as defined above. However, the use of derivatives or variants is also envisaged, for example to study the effect of mutations in the amino acid sequence on the function of the polypeptides.

In such assays, one or both of the binding domain, sequence or polypeptide and its binding partner typically comprises a detectable label including, but not exclusively, a fluorescent or other light-emitting label, which may for example be a polypeptide linked to the binding domain or binding partner. By measuring changes in signal emission during a physiological process, such as a response to a biologically active compound, the extent of association can be determined. Where this association is dependent on a post-translational modification, the activity of the cellular enzyme responsible for the modification can also be determined. An important feature of the invention is that such measurements (e.g., of a shift in FRET or other signal emitted by a detectable label) can be performed in real-time. This allows for sensitive assessment of reaction kinetics based upon the rate of change of the protein-binding-dependent signal emission or absorption by the label(s).

As used herein, the term "binding domain" in a three-dimensional sense refers to the amino acid residues of a first polypeptide required for modification-dependent binding between the first polypeptide and its binding partner. The amino acids of a "binding domain" may be either contiguous or non-contiguous and may form a binding pocket for modification-dependent binding. A binding domain typically includes at least 6 or more, preferably 10 or more, amino acids which are contiguous or non-contiguous, but are necessary for modification-dependent binding to the binding partner, and may include a full-length protein.

A binding domain which is of use in the invention is typically a "natural binding domain", i.e. a binding domain that exists in nature. In particular, the binding domain preferably exhibits modification-dependent binding to a binding partner in nature. A binding domain of use in the invention may be used as a discrete polypeptide domain or may be present in the context of a larger polypeptide molecule (i.e., one which comprises amino acids other than those of the natural binding domain) such as in its natural polypeptide context.

As used herein with regard to modification of a polypeptide, the terms "site" and "site sufficient for the addition of" refer to an amino acid sequence which is recognized by (i.e., a signal for) a modifying enzyme for the purpose of post-translational modification (i.e., addition or removal of a "moiety" as defined below) of the polypeptide or a portion thereof. A "site" additionally refers to the single amino acid which is modified. It is contemplated that a site comprises a small number of amino acids, as few as one but typically from 2 to 10, less often up to 30 amino acids, and further that a site comprises fewer than the total number of amino acids present in the polypeptide.

A "site", for post-translational modification may be present on either or both of a binding domain and its binding partner, preferably on both. If such sites are present on both the binding domain and the binding partner, binding between the natural binding domain and its binding partner may be dependent upon the modification state of either one or both sites. Where sites are present on both the binding domain and the binding partner, the sites may be the same or different. The binding domain and/or binding partner may comprise more than one site. Again the sites may be the same or different.

If a single polypeptide chain comprises the binding domain and its binding partner (or two natural binding domains), the state of post-translational modification of one or both sites will determine whether binding between the two domains occurs.

As used herein, the term "modification" or "post-translational modification" typically refers to the addition or removal of a chemical "moiety", as described herein, to/from a site on a polypeptide chain. However, proteolytic cleavage of the binding domain and or partner is also preferably included within the meaning of these terms. Often, the post-translational modification is reversible, such that repeating cycles of addition and removal of a modifying moiety may be observed.

As used interchangeably herein, the terms "moiety" and "group" refer to one of the post-translationally added or removed groups referred to herein: i.e., one of a ubiquitin moiety, a glycosyl moiety, a fatty acyl moiety, a sentrin moiety, an ADP-ribosyl or a phosphate moiety.

As used herein, the term "binding partner" refers to a polypeptide or fragment thereof (a peptide) that binds to a binding domain, sequence or polypeptide, as defined herein, preferably in a manner which is dependent upon the state of modification of a site for post-translational modification which is, at a minimum, present upon the binding domain, sequence or polypeptide; the binding partner itself may, optionally, comprise such a site and binding between the binding domain, fragment or polypeptide with its corresponding binding partner may, optionally, depend upon modification of that site. A binding partner does not necessarily have to contain a site for post-translational modification if such a site is not required to be present on it for modification-dependent association between it and a binding domain, sequence or polypeptide. Binding partners of use in the invention are typically those which are found in nature, or derivatives thereof, and exhibit natural modification-dependent binding to a binding domain, sequence or polypeptide of the invention as defined herein. In one embodiment of the invention, a binding partner is shorter (i.e., by at least one N-terminal or C-terminal amino acid) than the natural full-length polypeptide.

As used herein, the term "associates" or "binds" refers to a binding domain as described herein and its binding partner having a binding constant sufficiently strong to allow detection of binding by FRET or other detection means, which are in physical contact with each other and therefore typically have a dissociation constant (Kd) of about 10 μM or lower. The contact region may include all or parts of the two molecules. Therefore, the terms "substantially dissociated" and "dissociated" or "substantially unbound" or "unbound" refer to the absence or loss of contact between such regions, such that the binding constant is reduced by an amount which produces a discernable change in a signal compared to the bound state, including a total absence or loss of contact, such that the proteins are completely separated, as well as a partial absence or loss of contact, so that the body of the proteins are no longer in close proximity to each other but may still be tethered together or otherwise loosely attached, and thus typically have a dissociation constant greater than 10 μM (Kd). In many cases, the Kd will be in the mM range. The terms "complex", "dimer", "multimer" and "oligomer" as used herein, refer to the binding domain and its binding partner in the associated or bound state. More than one molecule of each of the two or more proteins may be present in a complex, dimer, multimer or oligomer according to the methods of the invention.

As used herein, the term "binding sequence" refers to that portion of a polypeptide comprising at least 4 or 6 amino acids, preferably at least 10, 20, 100 or 1000 contiguous (i.e., covalently linked by peptide bonds) amino acid residues or even as many residues as are comprised by a full-length protein, that are sufficient for modification-dependent binding to a binding partner. A binding sequence may exist on a polypeptide molecule that consists solely of binding sequence amino acid residues or may, instead, be found in the context of a larger polypeptide chain (i.e., one that comprises amino acids other than those of the binding sequence).

As used herein in reference to those binding sequences that are of use in the invention, the term "natural binding sequence" refers to a binding sequence, as defined above, which consists of an amino acid sequence which is found in nature and which is naturally dependent upon the modification state of a site for post-translational modification found within it for binding to a binding partner. A "natural binding sequence" may be present either in isolation or in the context of a larger polypeptide molecule, which molecule may be naturally occurring or recombinant. If present, amino acids outside of the binding sequence may be either natural, i.e., from the same polypeptide sequence from which the fragment is derived, or non-natural, i.e., from another (different) polypeptide, or non-natural (a sequence that is not derived from any known polypeptide). A binding sequence and its binding partner will however generally exist on two different polypeptide chains.

However, the methods described here need not necessarily make use of only naturally occurring sequences, including allelic variants. Modifications may be made to the amino acid sequence of the binding domain and/or the binding partner, such as substitutions, deletions and/or insertions. In particular, such modifications may be made to identify important regions of the molecule with respect to, for example, protein-protein interactions.

As used herein, the term "binding polypeptide" refers to a molecule comprising multiple binding sequences, as defined above, which sequences are derived from a single, naturally-occurring polypeptide molecule and are both necessary and, in combination, sufficient to permit modification-state-dependent binding of the binding polypeptide to its binding partner, as defined above, wherein the sequences of the binding polypeptide are either contiguous or are non-contiguous. As used herein in reference to the component binding sequences of a binding polypeptide, the term "non-contiguous" refers to binding sequences which are linked by intervening naturally-occurring, as defined herein, amino acid sequences. The amino acids of a polypeptide that do not significantly contribute to the modification-state-dependent binding of that polypeptide to its binding partner may be those amino acids which are naturally present and link the binding sequences in a binding polypeptide or they may be derived from a different polypeptide. A binding polypeptide and its binding partner (which may, itself, be a binding domain, sequence or polypeptide, as defined herein will typically exist on two different polypeptide chains.

It is preferred that the binding domain and/or binding partner comprise a sequence which directs modification of a site on the binding domain and/or binding partner by one or more of the following enzymes: a carbohydrate transferase (e.g., a UDP-N-Acetylglucosamine-Dolichyl-phosphate-N-acetylsglucosaminephospho transferase or an O-Glc-NAc transferase), a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyl transferase (e.g., a glycylpeptide-N-tetradecanoyltransferase (peptide-N-myristoyltransferase)), an NAD:Arginine ADP ribosyltransferase, a protease, a protein kinase or a phosphatase.

It is additionally preferred that the site permits addition of a chemical moiety which may be, for example, a ubiquitin moiety, a glycosyl moiety, an ADP-ribosyl-moiety, a fatty acid moiety, a sentrin moiety or a phosphate group and the addition prevents binding of the binding domain to the binding partner.

As used herein the term "prevents binding" or "prevents association" refers to the ability of at least one of a ubiquitin moiety, a glycosyl moiety, a fatty acyl moiety, a sentrin moiety, an ADP-ribosyl moiety or a phosphate moiety to inhibit the association, as defined above, of a binding domain and a binding partner thereof by at least 10%, preferably by 25 to 50%, highly preferably by 75 to 90% and, most preferably, by 95-100% relative to the association observed in the absence of such a modification under the same experimental conditions.

According to another preferred embodiment, the site permits addition of a chemical moiety which may be for example: a ubiquitin moiety, a glycosyl moiety, an ADP-ribosyl moiety, a fatty acid moiety, a sentrin moiety or a phosphate moiety, and the addition promotes binding of the binding domain to the binding partner.

As used herein, the term "promotes binding" refers to that which causes an increase in binding of the natural binding domain and its binding partner of at least two-fold, preferably 10- to 20-fold, highly preferably 50- to 100-fold, more preferably from 200- to 1000-fold, and, most preferably, from 200 to 10,000-fold.

Preferably, the site permits removal of a chemical moiety which may be: a ubiquitin moiety, a glycosyl moiety, and ADP-ribosyl moiety, a fatty acid moiety, a sentrin moiety or a phosphate moiety, and the removal prevents binding of the binding domain to the binding partner.

It is preferred that the site permits removal of a chemical moiety which may be: a ubiquitin moiety, a glycosyl moiety, an ADP-ribosyl moiety, a fatty acid moiety, a sentrin moiety or a phosphate moiety, and the removal promotes binding of the isolated natural binding domain to the binding partner.

A binding domain useful in the present invention will comprise a binding site which will permit it to bind to other polypeptides (binding partners) to form a complex. Polypeptides are known to be able to associate in a number of ways, and domains which mediate polypeptide association are known in the art. For example, coiled coils, acid patches, zinc fingers, calcium hands, WD40 motifs, SH2/SH3 domains and leucine zippers are all polypeptide domains known to mediate protein-protein interactions, as are other domains known to those skilled in the art.

The binding domains and binding partners are typically provided as sets since they are to be used to detect the activity of two or more different enzymes. Thus, generally, a set of reporter polypeptides for use in the methods of the invention comprises at least a first binding domain and binding partner thereof for use in detecting a first enzyme and least a second binding domain and binding partner thereof for use in detecting a second enzyme. The assay method is not however limited to measuring the activity of two enzymes at the same time but may be used to measure a plurality of enzyme activities. Accordingly, the set may comprise a plurality of reporter polypeptide pairs suitably chosen so that the activity of each enzyme of interest may be discriminated.

Polypeptides for use as reporters may be produced by a variety of techniques known in the art such as solid phase synthesis or recombinant expression. Such polypeptides may optionally include residues or chemical moieties to assist in immobilising one or more reporter polypeptide to a solid phase. For example, reporter polypeptides may be fusion polypeptides which comprise a heterologous sequence fused by recombinant techniques to the remainder of the polypeptide. Examples of fusion polypeptides include a hexahistidine tag, GAL4, beta-galactosidase and epitope tags Reporter polypeptides may also comprise a detectable label as set out below.

In a preferred *in vitro* embodiment, at least one of the binding domain or binding partner is immobilised to a solid support. Suitable supports include beads, "chips", resins, matrices, gels, and the material forming the walls of a vessel. Matrices, and in particular gels, such as agarose gels, may conveniently be packed into columns. A particular advantage of solid phase immobilisation is that the reagents may be removed from contact with the polypeptide(s) with facility.

Sets of reporter polypeptides may be immobilised to solid substrates to produce peptide arrays. The reporter polypeptides are typically immobilised onto or in discrete regions of a solid substrate. The substrate may be porous to allow immobilisation within the substrate or substantially non-porous, in which case the sensing elements are typically immobilised on the surface of the substrate. The solid substrate may be made of any material to which polypeptides can bind, either directly or indirectly. Examples of suitable solid substrates include flat glass, silicon wafers, mica, ceramics and organic polymers such as plastics, including polystyrene and polymethacrylate. It may also be possible to use semi-permeable membranes such as nitrocellulose or nylon membranes, which are widely available. The semi-permeable membranes may be mounted on a more robust solid surface such as glass. The surfaces may optionally be coated with a layer of metal, such as gold, platinum or other transition metal. A particular example of a suitable solid substrate is the commercially available BIACore™ chip (Pharmacia Biosensors). A further example is a microtitre plate or similar configuration.

The solid substrate is conveniently divided up into sections. This may be achieved by techniques such as photoetching or by the application of hydrophobic inks, for example teflon-based inks (Cel-line, USA).

Attachment of the reporter polypeptides to the substrate may be by covalent or non-covalent means. The reporter polypeptides may be attached to the substrate via a layer of molecules to which the sensing elements bind. For example, the sensing elements may be labelled with biotin and the substrate coated with avidin and/or streptavidin. A convenient feature of using biotinylated sensing elements is that the efficiency of coupling to the solid substrate can be determined easily. Since the sensing elements may bind only poorly to some solid substrates, it is often necessary to provide a chemical interface between the solid substrate (such as in the case of glass) and the sensing element. Examples of suitable chemical interfaces include organofunctional silanes and long-chain thiol alkanes with terminal activatable groups such as terminal carboxylic acid groups. Another example is the use of polylysine coated glass, the polylysine then being chemically modified using standard procedures to introduce an affinity ligand such as nitrilotriacetate (NTA). Other methods for attaching molecules to the surfaces of sensor chips by the use of coupling agents are known in the art, see for example WO98/49557.

It is desirable to confirm the efficiency of coupling using standard techniques to establish the amount of sensing element bound at each cell on the solid substrate. This information may be used to normalise the results obtained from various positions in the array.

By way of an example, a suitable protocol involves the use of hexahistidine tagged polypeptides immobilised to microtitre plates. The Nitrillotriacetic acid ligand is bound to the plate surface according to manufacturer's instructions. Initially the plate is washed with PBS buffer containing 50 µM EDTA (enough to remove contaminating metal ions without stripping the $Ni^{2+}$) and then the plates are charged with $Ni^{2+}$ (100 µM in PBS buffer). Peptides (0.01-100 mM in PBS buffer) are added to the microplate wells (the capacity of binding is determined by the degree of derivatisation of Ni-NTA) and incubated for 1 to 2 hours at room temperature or overnight at 4°C. The unbound peptide is then removed by aspiration followed by a thorough wash with excess buffer. Excess wash buffer is removed by gentle tapping of the inverted plates on paper towels.

B. Detectable labels

In a particularly preferred embodiment, at least one, preferably both, of the binding domain and the binding partner comprise a detectable label. The label may be optical, for example fluorescent, an absorptive coloured particle or a dye, radioactive, such as may be applicable for use in a scintillation proximity assay, or a scintillation emulsion, enzymatic, such as a glucose oxidase sensor or other redox system or an immobilised chemical cascade, or based on mass, as might be applicable in a surface plasmon resonance-based assay, in which case either the protein or the binding partner must be of high molecular weight. Preferably, the label is fluorescent. Fluorescent labels are described in detail below

"Detectable label" in the context of the present invention means a moiety, proteinaceous or otherwise whose physical characteristics alter in a detectable manner depending on its molecular context, such as when the polypeptide to which it is linked binds to another polypeptide. It is particularly preferred that the detectable label is detectable in a non-invasive manner that does not require destruction of cellular material. Thus for example labels that do not require the extraction and resolution of cellular components and allow in situ measurements are preferred.

In one embodiment, the detectable label may comprise subunits or fragments of enzymes that are functional when associated. For example, β-galactosidase may be cleaved into two fragments ($\Delta\alpha$ and $\Delta\omega$) which when expressed together form a functional enzyme (see Sambrook *et al.*, ibid). Such a system has been used in intact yeast to monitor protein-protein interactions (Rossi *et al.*, 1997, Proc. Natl. Acad. Sci. USA 94: 8405-8410). Also, bacterial luciferase can be produced as two fragments which when expressed together and associate, form a functional enzyme (Almashanu *et al.*, 1996, Protein Eng. 9: 803-9).

Detectable labels also include moieties that may serve to increase the size of the reporter polypeptide for use in detection techniques that measure an increase in the size of a complex. Thus for example, a suitable moiety includes streptavidin which is attached to the reporter polypeptide via a biotin moiety. Biotinylation of peptide is well known in the art. A biotin/streptavidin moiety is described in the Examples where the measurement technique used was fluorescent polarisation which measures the rate of tumbling of macromolecules. When the reporter polypeptide coupled to biotin/streptavidin binds to a fluorescently labelled binding partner, the polarisation value of the fluorescent labelled binding partner changes as a result of the large increase in size of the complex.

Since in the assays of the present invention, two or more enzyme activities are measured, it is important to select the detectable labels such that the signal generated by the reporter polypeptides responsive to one enzyme of interest can be distinguished from the signal or signals generated by the reporter polypeptides responsive to any other enzymes of interest.

Where, for example, the binding domain-label and binding partner-label polypeptides will be expressed by living cells such as in transgenic organisms from a construct present in the genome of the organism, the label will be in the form of a polypeptide whose amino acid constituents are naturally occurring amino acids, including those arising from post-translational modifications. The nucleic acid constructs encoding said polypeptides may be introduced into the genome of the organism using standard techniques. Typically, the binding domain and binding partner is linked to the detectable label by being expressed in the form of a fusion protein. However, the binding domain and/or binding partner may be linked to their detectable label by any physiological applicable means. For example they may be part of a multi-protein complex joined by disulphide bonding or by non-covalent bonding, such as electrostatic interactions, hydrophobic interactions and/or van der Waal's forces.

Preferably, the changing physical characteristics may be detected in living cells using non-invasive techniques such as FRET. Thus in a preferred embodiment, the detectable label is a fluorescent protein.

"Fluorescent protein" refers to any protein which fluoresces when excited with appropriate electromagnetic radiation. This includes proteins whose amino acid sequences are either natural or engineered. A "fluorescent protein" is a full-length fluorescent protein or fluorescent fragment thereof.

However, non-proteinaceous fluorescent labels may be used, especially in *in vitro* assay systems. The choice of fluorescent label will be such that upon excitation with light, labelled peptides which are associated will show optimal energy transfer between fluorophores. In the presence of a protein modifying enzyme that recognizes the site for protein modification present on the natural binding domain and, optionally, the binding partner, the natural binding domain and its binding partner dissociate due to a structural or electrostatic change which occurs as a consequence of addition or removal of a chemical moiety, as described herein, to/from the enzyme recognition site, thereby leading to a decrease in energy transfer and increased emission of light by the donor fluorophore. In this way, the state of polypeptide modification can be monitored and quantitated in real-time.

As used herein, the terms "fluorophore" and "fluorochrome" refer interchangeably to a molecule which is capable of absorbing energy at a wavelength range and releasing energy at a wavelength range other than the absorbance range. The term "excitation wavelength" refers to the range of wavelengths at which a fluorophore absorbs energy. The term "emission wavelength" refers to the range of wavelength that the fluorophore releases energy or fluoresces.

A non-limiting list of chemical fluorophores of use in the invention, along with their excitation and emission wavelengths, is presented in Table 1.

Table 1

| Fluorophore | Excitation (nm) | Emission (nm) | Color |
|---|---|---|---|
| PKH2 | 490 | 504 | green |
| PKH67 | 490 | 502 | green |
| Fluorescein (FITC) | 495 | 525 | green |
| Hoechst 33258 | 360 | 470 | blue |
| R-Phycoerythrin (PE) | 488 | 578 | orange-red |
| Rhodamine (TRITC) | 552 | 570 | red |
| Quantum Red™ | 488 | 670 | red |
| PKH26 | 551 | 567 | red |
| Texas Red | 596 | 620 | red |
| Cy3 | 552 | 570 | red |

It should also be noted that where the fluorescent technique used requires both a donor molecule and an acceptor molecule, naturally occurring or engineered tryptophan residues within the binding domain and/or partner are suitable as a donor and therefore in this situation, only one of the molecules need be labelled with a fluorescent molecule.

Examples of fluorescent proteins which vary among themselves in excitation and emission maxima are listed in Table 1 of WO 97/28261. These (each followed by [excitation max./emission max.] wavelengths expressed in nanometers) include wild-type Green Fluorescent Protein [395(475)/508] and the cloned mutant of Green Fluorescent Protein variants P4 [383/447], P4-3 [381/445], W7 [433(453)/475(501)], W2 [432(453)/480], S65T [489/511], P4-1 [504 (396)/480], S65A [471/504], S65C [479/507], S65L [484/510], Y66F [360/442], Y66W [458/480], 10c [513/527], W1B [432(453)/476(503)], Emerald [487/508] and Sapphire [395/511]. This list is not exhaustive of fluorescent proteins known in the art; additional examples are found in the Genbank and SwissProt public databases. Further examples are described in Matz *et al*., 1999 (Nature Biotech 17: 969-973) and include red fluorescent protein from *Discosoma sp.* (drFP583).

When used in a FRET assay, the fluorescent labels are chosen such that the excitation spectrum of one of the labels (the acceptor label) overlaps with the emission spectrum of the excited fluorescent label (the donor label). The donor label is excited by light of appropriate intensity within the donor's excitation spectrum. The donor then emits some of the absorbed energy as fluorescent light and dissipates some of the energy by FRET to the acceptor fluorescent label. The fluorescent energy it produces is quenched by the acceptor fluorescent label. FRET can be manifested as a reduction in the intensity of the fluorescent signal from the donor, reduction in the lifetime of its excited state, and re-emission of fluorescent light at the longer wavelengths (lower energies) characteristic of the acceptor. When the donor and acceptor labels become spatially separated, FRET is diminished or eliminated.

One can take advantage of the FRET exhibited by a binding domain and its binding partner labelled with different fluorescent labels, wherein one is linked to a donor and the other to an acceptor label, in monitoring protein-protein interactions according to the present invention. A single polypeptide may comprise a blue fluorescent donor label and a green fluorescent acceptor label, wherein each is fused to a different assay component (i.e., in which one is fused to the binding domain and the other to its binding partner); such a construct is herein referred to as a "tandem" fusion protein.

Alternatively, two distinct polypeptides ("single" fusion proteins) one comprising or a natural binding domain and the other its binding partner may be differentially labelled with the donor and acceptor fluorescent protein labels, respectively. The construction and use of tandem fusion proteins in the invention can reduce significantly the molar concentration of peptides necessary to effect an association between differentially-labelled polypeptide assay components relative to that required when single fusion proteins are instead used. The labelled binding domain, sequence or polypeptide and/or its binding partner may typically be produced using chemical synthesis techniques such as solid phase synthesis or the expression of recombinant nucleic acid molecules comprising an in-frame fusion of sequences encoding such a polypeptide and a protein label *in vitro* or *in vivo*, for example in a transgenic animal.

A recombinant nucleic acid molecule of use in the invention may be constructed and expressed by molecular methods well known in the art, and may additionally comprise sequences including, but not limited to, those which encode a tag (e.g., a histidine tag) to enable easy purification, a secretion signal, a nuclear localization signal or other primary sequence signal capable of targeting the construct to a particular cellular location, if it is so desired. Nucleic acid sequences may, for example, be modified by virtue of the degeneracy of the genetic code to optimise the codon usage to take into account the codon bias in a given transgenic organism of interest.

The means by which a binding domain and its binding partner are assayed for association using fluorescent protein labels according to the invention may be briefly summarized as follows:

Whether or not the natural binding domain and its binding partner are present on a single polypeptide molecule, one maybe labelled with a green fluorescent protein, while the other is preferably labelled with a red or, alternatively, a blue fluorescent protein. Useful donor:acceptor pairs of fluorescent proteins (see WO97/28261) include, but are not limited to:

Donor:    S72A, K79R, Y145F, M153A and T203I (excitation 395nm; emission 511)

Acceptor: S65G, S72A, K79R and T203Y (excitation 514 nm; emission 527 nm), or T203Y/S65G, V68L, Q69K or S72A (excitation 515nm; emission 527nm).

An example of a blue:green pairing is P4-3 (shown in Table 1 of WO97/28261) as the donor label and S65C (also of Table 1 of WO97/28261) as the acceptor label. The natural binding domain, sequence or polypeptide and corresponding binding partner are exposed to light at, for example, 368 nm, a wavelength that is near the excitation maximum of P4-3. This wavelength excites S65C only minimally. Upon excitation, some portion of the energy absorbed by the blue fluorescent protein label is transferred to the acceptor label through FRET if the natural binding domain, sequence or polypeptide and its binding partner are in close association. As a result of this quenching, the blue fluorescent light emitted by the blue fluorescent protein is less bright than would be expected if the blue fluorescent protein existed in isolation. The acceptor label (S65C) may re-emit the energy at longer wavelength, in this case, green fluorescent light.

By way of an example, after an event, such as an intracellular event, resulting in modification of one or both of the natural binding domain and its binding partner by a protein modifying enzyme, the natural binding domain and its binding partner (and, hence, the green and red or, less preferably, green and blue fluorescent proteins) physically separate or associate, accordingly inhibiting or promoting FRET. For example, if activity of the modifying enzyme results in dissociation of a protein:protein complex, the intensity of visible blue fluorescent light emitted by the blue fluorescent protein increases, while the intensity of visible green light emitted by the green fluorescent protein as a result of FRET, decreases.

Such a system is useful to monitor the activity of enzymes that modify a site for post-translational modification of a binding domain and, optionally, its binding partner to which the fluorescent protein labels are fused as well as the activity of protein modifying enzymes or candidate modulators thereof.

Advantages of single- and tandem fluorescent protein/polypeptides comprising a binding domain fused to a fluorescent protein include the greater extinction coefficient and quantum yield of many of these proteins compared with those of the non-peptide fluorophores.

Thus, in a preferred embodiment, the enzyme of interest's substrate (i.e., the binding domain and, optionally, the corresponding binding partner), and reaction products (i.e., the binding domain and, optionally, the corresponding binding partner after modification) are both fluorescent but with different fluorescent characteristics.

In particular, the substrate and modified products exhibit different ratios between the amount of light emitted by the donor and acceptor labels. Therefore, the ratio between the two fluorescences measures the degree of conversion of substrate to products, independent of the absolute amount of either, the optical thickness of the sample, the brightness of the excitation lamp, the sensitivity of the detector, etc. Furthermore, *Aequorea*-derived or - related fluorescent protein labels tend to be protease resistant. Therefore, they are likely to retain their fluorescent properties throughout the course of an experiment.

As described above, the donor fluorescent protein label is capable of absorbing a photon and transferring energy to another fluorescent label. The acceptor fluorescent protein label is capable of absorbing energy and emitting a photon. If needed, the linker connects the natural binding domain and its binding partner either directly or indirectly, through an intermediary linkage with one or both of the donor and acceptor fluorescent protein labels. Regardless of the relative order of the binding domain, its binding partner and the donor and acceptor fluorescent protein labels on a polypeptide molecule, it is essential that sufficient distance be placed between the donor and acceptor by the linker and/or the binding domain and its binding partner to ensure that FRET does not occur unless the binding domain and its binding partner bind. It is desirable, as described in greater detail in WO97/28261, to select a donor fluorescent protein label with an emission spectrum that overlaps with the excitation spectrum of an acceptor fluorescent protein label. In some embodiments of the invention the overlap in emission and excitation spectra will facilitate FRET. A fluorescent protein of use in the invention includes, in addition to those with intrinsic fluorescent properties, proteins that fluoresce due intramolecular rearrangements or the addition of cofactors that promote fluorescence.

For example, green fluorescent proteins ("GFPs") of cnidarians, which act as their energy-transfer acceptors in bioluminescence, can be used in the invention. A green fluorescent protein, as used herein, is a protein that fluoresces green light, and a blue fluorescent protein is a protein that fluoresces blue light. GFPs have been isolated from the Pacific Northwest jellyfish, *Aequorea victoria*, from the sea pansy, *Renilla reniformis,* and from *Phialidium gregarium*. (Ward *et al.*, 1982, Photochem. Photobiol., 35: 803-808; Levine *et al.*, 1982, Comp. Biochem. Physiol., 72B: 77-85). See also Matz, *et al.*, 1999, ibid for fluorescent proteins isolated recently from Anthoza species (accession nos. AF168419, AF168420, AF168421, AF168422, AF168423 and AF168424).

A variety of *Aequorea*-related GFPs having useful excitation and emission spectra have been engineered by modifying the amino acid sequence of a naturally occurring GFP from Aequorea victoria. (Prasher *et al.*, 1992, Gene, 111: 229-233; Heim *et al.*, 1994, Proc. Natl. Acad. Sci. U.S.A., 91: 12501-12504; PCT/US95/14692). As used herein, a fluorescent protein is an Aequorea-related fluorescent protein if any contiguous sequence of 150 amino acids of the fluorescent protein has at least 85% sequence identity with an amino acid sequence, either contiguous or non-contiguous, from the wild-type Aequorea green fluorescent protein (SwissProt Accession No. P42212). More preferably, a fluorescent protein is an Aequorea-related fluorescent protein if any contiguous sequence of 200 amino acids of the fluorescent protein has at least 95% sequence identity with an amino acid sequence, either contiguous or non-contiguous, from the wild type *Aequorea* green fluorescent protein of SwissProt Accession No. P42212. Similarly, the fluorescent protein may be related to *Renilla* or *Phialidium* wild-type fluorescent proteins using the same standards.

*Aequorea*-related fluorescent proteins include, for example, wild-type (native) *Aequorea victoria* GFP, whose nucleotide and deduced amino acid sequences are presented in Genbank Accession Nos. L29345, M62654, M62653 and others *Aequorea*-related engineered versions of Green Fluorescent Protein, of which some are listed above. Several of these, i.e., P4, P4-3, W7 and W2 fluoresce at a distinctly shorter wavelength than wild type.

C. Production of polypeptides of use in the invention

A polypeptide consisting of- or comprising a natural binding domain or a binding partner thereof may be synthesized by Fmoc or Tboc chemistry according to methods known in the art (e.g., see Atherton et al., 1981, *J.*

*Chem. Soc. Perkin I*, 1981(2): 538-546; Merrifield, 1963, *J. Am. Chem. Soc.*, 85: 2149-2154, respectively). Following deprotection and cleavage from the resin, peptides are desalted by gel filtration chromatography and analyzed by mass spectroscopy, HPLC, Edman degradation and/or other methods as are known in the art for protein sequencing using standard methodologies.

Alternatively, nucleic acid sequences encoding such proteins may be expressed either in cells, including the cells of an intact multicellular organism or in an *in vitro* transcription/translation system. Optionally, the proteins may be purified by methods well known in the art.

Labelling a natural binding domain and/or a binding partner therefor with a detectable label

Many amino acid residues have chemistry allowing labelling with commercially available fluorescent or other labels. The most useful of these are those with ionisable side chains aspartic acid, glutamic acid, lysine, arginine, cysteine, histidine and tyrosine. The labelling reagent may be a group conferring the desired property such as fluorescence, and preferably includes a group involved in the conjugation of said label to the target polypeptide. The most commonly used functional groups in this context are those which react with amines by either acylation or alkylation. These include isothiocyanates, isocyanates, acyl azides, NHS esters and many others. Also common is the use of thiol-directed groups such as haloacetates and maleimides. These label primarily at the free sulfhydryl group of cysteine residues.

A number of protocols have been devised to achieve labelling at a specific site in a synthesised peptide . These are well known to those skilled in the art, and many are detailed in Bioconjugate Techniques, G.T.Hermanson, Academic Press 1996. It is possible to bias reactions to achieve specific labelling on one functional group and to reduce promiscuous reaction of the label with other sites in the polypeptide. Alternatively, labelling of the molecule may be concurrent with synthesis. This may be achieved either by the use of a labelled amino acid in the synthesis process or by the specific deprotection and labelling of the residue of interest before deprotection of other potentially reactive residues at the completion of the synthesis.

By way of example, a natural binding domain or binding partner therefor may be labelled with thiol reactive derivatives of fluorescein and tetramethylrhodamine (isothiocyanate or iodoacetamide derivatives, Molecular Probes, Eugene, OR, USA) using procedures described by Hermanson G.T., 1996, *Bioconjugate Techniques,* Academic Press, London. Alternatively, primary-amine-directed conjugation reactions can be used to label lysine sidechains or the free peptide N-terminus (Hermason, 1996, supra). Fluorescent peptides are separated from unreacted fluorophores by gel filtration chromatography or reverse phase HPLC or equilibrium dialysis.

Recombinant expression of reporter polypeptides - Reporter polypeptide constructs

As stated above, recombinant nucleic acid constructs of particular use in the invention are typically those which comprise in-frame fusions of sequences encoding a binding domain or a binding partner therefor and a label protein, such as a fluorescent protein. By way of example, if a fluorescently labelled binding domain and its binding partner are to be expressed as part of a single polypeptide, the nucleic acid molecule additionally encodes, at a minimum, a donor fluorescent protein label fused to one, an acceptor fluorescent protein label fused to the other, a linker that couples the two and is of sufficient length and flexibility to allow for folding of the polypeptide and pairing of the natural binding domain, sequence or polypeptide with the binding partner, and gene regulatory sequences operatively linked to the fusion coding sequence. If single fusion proteins are instead encoded (whether by one or more nucleic acid molecules), each nucleic acid molecule need only encode a binding domain or a binding partner therefor, fused either to a donor or acceptor fluorescent protein label and operatively linked to gene regulatory sequences.

Preferably, the fusion protein comprising the binding domain and the fusion protein comprising the binding partner are encoded by separate nucleic acid constructs.

"Operatively-linked" refers to polynucleotide sequences which are necessary to effect the expression of coding and non-coding sequences to which they are ligated. The nature of such control sequences differs depending upon the host organism; in prokaryotes, such control sequences generally include promoter, ribosomal binding site, and transcription termination sequence; in eukaryotes, generally, such control sequences include promoters and a transcription termination sequence. The term "control sequences" is intended to include, at a minimum, components whose presence can influence expression, and can also include additional components whose presence is advantageous, for example, leader sequences and fusion partner sequences.

Particularly preferred regulatory sequences are those that confer tissue-specific or inducible expression. Tissue-specific expression may be used to confine expression of the binding domain and/or binding partner to a cell type or tissue/organ of interest. Inducible expression allows the researcher to control when expression of the polypeptides takes place. Thus, for example, a transgenic organism may be produced and cross-bred in the absence of expression of the binding domain and/or binding partner, and only when it is desired to monitor the interaction between the binding domain and binding partner is expression induced, by for example administering a compound that upregulates expression from the inducible regulatory construct.

Where the nucleic acid constructs are to be integrated into the host genome, it is important to include sequences that will permit expression of polypeptides in a particular genomic context. One possible approach would be to use homologous recombination to replace the endogenous gene encoding the binding protein or binding partner with a sequence encoding the corresponding polypeptide fused to a detectable label. This should ensure that the fusion polypeptide is subject to the same transcriptional regulatory mechanisms as the endogenous gene. Alternatively, homologous recombination may be used in a similar manner but with the regulatory sequences also replaced so that the fusion polypeptide is subject to a different form of regulation - such as inducible expression in the presence of an exogenously added compound.

However, if the construct is placed elsewhere in the genome, it is possible that the chromatin in that region will be transcriptionally silent and in a condensed state. If this occurs, then the labelled polypeptide will not be expressed - these are termed position-dependent effects. To overcome this problem, it may be desirable to include locus control regions (LCRs) that maintain the intervening chromatin in a transcriptionally competent open conformation. LCRs (also known as scaffold attachment regions (SARs) or matrix attachment regions (MARs)) are well known in the art - an example being the chicken lysozyme A element (Stief *et al*., 1989, Nature 341: 343), which can be positioned around an expressible gene of interest to effect an increase in overall expression of the gene and diminish position dependent effects upon incorporation into the organism's genome (Stief *et al*., 1989, supra). Another example is the CD2 gene LCR described by Lang *et al*., 1991, Nucl. Acid. Res. 19: 5851-5856.

D. Methods by which to detect protein:protein binding in assays of the invention

According to the invention, the association of the binding domain and binding partner is measured using detection techniques that can determine the formation or destruction of protein:protein complexes. Assays may be carried out *in vitro*, in whole cell assay formats or *in vivo*. *In vitro* assays and whole cell assays may advantageously be used in high throughput screens when seeking to identifying modulators of enzyme activity. An important aspect of the present invention is that the activity of more than one enzyme in a given system can be measured simultaneously. Thus it is possible to measure the activity of two or more different enzyme simultaneously in the same cell or reaction vessel.

Methods of detection without use of label are known in the art. These include detection using surface plasmon resonance to detect changes in the mass of the immobilised polypeptide, which would occur if binding of the partner polypeptide increased or decreased. Such measurements may be made for example using a BIAcore machine. Alternatively, mass spectroscopy may be used to detect the presence and mass of a bound species interacting with an immobilised peptide.

However, it is preferred to use labelled binding domains and binding partners and therefore techniques which measure physical parameters arising from such labels are typically used. Of particular use in the invention are those methods which entail fluorescent labeling of the binding domain and/or its binding partner, and subsequent detection of changes in fluorescence, whether in frequency or level, when the binding domain interacts with its binding partner. Several such procedures are briefly summarized below.

A number of parameters of fluorescence output are envisaged including

1. measuring fluorescence emitted at the emission wavelength of the acceptor (A) and donor (D) and determining the extent of energy transfer by the ratio of their emission amplitudes;
2. measuring the fluorescence lifetime of D;
3. measuring the rate of photobleaching of D;
4. measuring the anisotropy of D and/or A. or
5. measuring the Stokes shift monomer; excimer fluorescence.

Fluorescent resonance energy transfer (FRET)

A tool with which to assess the distance between one molecule and another (whether protein or nucleic acid) or between two positions on the same molecule is provided by the technique of fluorescent resonance energy transfer (FRET), which is now widely known in the art (for a review, see Matyus, 1992, J. Photochem. Photobiol. B: Biol., 12: 323-337). FRET is a radiationless process in which energy is transferred from an excited donor molecule to an acceptor molecule; the efficiency of this transfer is dependent upon the distance between the donor and acceptor molecules, as described below. Since the rate of energy transfer is inversely proportional to the sixth power of the distance between the donor and acceptor, the energy transfer efficiency is extremely sensitive to distance changes. Energy transfer is said to occur with detectable efficiency in the 1-10 nm distance range, but is typically 4-6 nm for favorable pairs of donor and acceptor.

Radiationless energy transfer is based on the biophysical properties of fluorophores. These principles are reviewed elsewhere (Lakowicz, 1983, Principles of Fluorescence Spectroscopy, Plenum Press, New York; Jovin and Jovin, 1989, Cell Structure and Function by Microspectrofluorometry, eds. E. Kohen and J.G. Hirschberg, Academic Press). Briefly, a fluorophore absorbs light energy at a characteristic wavelength. This wavelength is also known as the excitation wavelength. The energy absorbed by a flurochrome is subsequently released through various pathways, one being emission of photons to produce fluorescence. The wavelength of light being emitted is known as the emission wavelength and is an inherent characteristic of a particular fluorophore. Radiationless energy transfer is the quantum-mechanical process by which the energy of the excited state of one fluorophore is transferred without actual photon emission to a second fluorophore. That energy may then be subsequently released at the emission wavelength of the second fluorophore. The first fluorophore is generally termed the donor (D) and has an excited state of higher energy than that of the second fluorophore, termed the acceptor (A). The essential features of the process are that the emission specturm of the donor overlap with the excitation spectrum of the acceptor, and that the donor and acceptor be sufficiently close. The distance over which radiationless energy transfer is effective depends on many factors including the fluorescence quantum efficiency of the donor, the extinction coefficient of the acceptor, the degree of overlap of their respective spectra, the refractive index of the medium, and the relative orientation of the transition moments of the two fluorophores. In addition to having an optimum emission range overlapping the excitation wavelength of the other fluorophore, the distance between D and A must be sufficiently small to allow the radiationless transfer of energy between the fluorophores.

FRET may be performed either *in vivo* or *in vitro*. According to the invention, a binding domain, sequence or polypeptide and its binding partner, comprised either by the same or by different polypeptide molecules, are differentially labelled, one with a donor and the other with an acceptor, and differences in fluorescence under different conditions measured using a fluorimeter or laser-scanning microscope. It will be apparent to those skilled in the art that excitation/detection means can be augmented by the incorporation of photomultiplier means to enhance detection sensitivity. The differential labels may comprise either two different fluorescent labels or a fluorescent label and a molecule known to quench its signal; differences in the proximity of the natural binding domain to its binding partner under different conditions can be gauged based upon a difference in the fluorescence spectrum or intensity observed.

This combination of protein-labeling methods and devices confers a distinct advantage over prior art methods for determining the association of polypeptides, optionally dependent on the activity of protein-modifying enzymes, as described above, in that results of all measurements are observed in real time (i.e., as a reaction progresses). This is significantly advantageous, as it allows both for rapid data collection and yields information regarding reaction kinetics under various conditions.

A sample assayed according to the invention therefore comprises a mixture at equilibrium of the labelled natural binding domain and its binding partner which, when disassociated from one another, fluoresce at one frequency and, when complexed together, fluoresce at another frequency or, alternatively, of molecules which either do or do not fluoresce depending upon whether or not they are associated.

The binding domain and/or binding partner thereof is typically modified so as to contain a chemical fluorophore or fused in-frame with a fluorescent protein, as described below. The choice of fluorescent label will be such that upon excitation with light, labelled peptides which are associated will show optimal energy transfer between fluorophores. In the presence of a protein modifying enzyme that recognizes the site for protein modification present on the natural binding domain and, optionally, the binding partner, the natural binding domain and its binding partner dissociate due to a structural or electrostatic change which occurs as a consequence of addition or removal of a chemical moiety, as described herein, to/from the enzyme recognition site, thereby leading to a decrease in energy transfer and increased emission of light by the donor fluorophore. In this way, the state of polypeptide association/modification can be monitored and quantitated in real-time.

Alternative fluorescent techniques suitable for monitoring protein-protein binding in assays of the invention

One embodiment of the technology can utilize monomer-excimer fluorescence as the output.

The fluorphore pyrene when present as a single copy displays fluorescent emission of a particular wavelength significantly shorter than when two copies of pyrene form a planar dimer (excimer), as depicted. As above, excitation at a single wavelength (probably 340nm) is used to review the excimer fluorescence (~470nm) over monomer fluorescence (~375nm) to quantify assembly:disassembly of the reporter molecule.

Additional embodiments of the present invention are not dependent on FRET. For example the invention can make use of fluorescence correlation spectroscopy (FCS), which relies on the measurement of the rate of diffusion of a label (see Elson and Magde, 1974 Biopolymers, 13: 1-27; Riger *et al*., 1992, in Fluorescence Spectroscopy: New Methods and Applications; Springer Verlag, pp.13-24; Eigen and Rigler, 1994, Proc. Natl. Acad. Sci. U.S.A., 91: 5740-5747; Kinja and Rigler, 1995, Nucleic Acids Res., 23: 1795-1799).

In FCS, a focused laser beam illuminates a very small volume of solution, of the order of $10^{-15}$ liter, which at any given point in time contains only one molecule of the many under analysis. The diffusion of single molecules through the illuminated volume, over time, results in bursts of fluorescent light as the labels of the molecules are excited by the laser. Each individual burst, resulting from a single molecule, can be registered.

A labelled polypeptide will diffuse at a slower rate if it is large than if it is small. Thus, multimerized polypeptides will display slow diffusion rates, resulting in a lower number of fluorescent bursts in any given timeframe, while labelled polypeptides which are not multimerized or which have dissociated from a multimer will diffuse more rapidly. Binding of polypeptides can be calculated directly from the diffusion rates through the illuminated volume.

Where FCS is employed, rather than FRET, it is not necessary to label more than one polypeptide. Preferably, a single polypeptide member of the multimer is labelled. The labelled polypeptide dissociates from the multimer as a result of modification, thus altering the FCS reading for the fluorescent label.

A further detection technique which may be employed in the method of the present invention is the measurement of time-dependent decay of fluorescence anisotropy. This is described, for example, in Lacowicz, 1983, Principles of Fluorescence Spectroscopy, Plenum Press, New York, incorporated herein by reference (see, for example, page 167).

Fluorescence anisotropy relies on the measurement of the rotation of fluorescent groups. Larger multimers of polypeptides rotate more slowly than monomers, allowing the formation of multimers to be monitored.

Another well known technique is fluorescence polarisation (Lynch *et al*., 1999, Analytical Biochemistry 275: 62-67; Pin *et al*., 1999, Analytical Biochemistry 275: 156-161 and references contained therein), which is described in the Examples.

Non-fluorescent methods to detect protein:protein binding according to the invention

The invention may be configured to exploit a number of non-fluorescent labels. In a first embodiment, the binding domain and binding partner therefor form, when bound, an active enzyme which is capable of participating in an enzyme-substrate reaction which has a detectable endpoint. The enzyme may comprise two or more polypeptide chains or regions of a single chain, such that upon binding of the binding domain to the binding partner, which are present either on two different polypeptide chains or in two different regions of a single polypeptide, these components assemble to form a functional enzyme. Enzyme function may be assessed by a number of methods, including scintillation counting and photospectroscopy. In a further embodiment, the invention may be configured such that the label is a redox enzyme, for example glucose oxidase, and the signal generated by the label is an electrical signal.

Modification of the binding domain and, optionally, its binding partner according to the invention is required to inhibit binding and, consequently, enzyme component assembly, thus reducing enzyme activity.

In another assay format, an enzyme is used together with a modulator of enzyme activity, such as an inhibitor or a cofactor. In such an assay, one of the enzyme and the inhibitor or cofactor is a natural binding domain, the other its binding partner. Binding of the enzyme to its inhibitor or cofactor results in modulation of enzymatic activity, which is detectable by conventional means (such as monitoring for the conversion of substrate to product for a given enzyme).

An example of a BIAcore assay system using immobilised peptides is described below

A Ni-NTA derivatised sensor chip (the chip is commercially available from BIAcore, Uppsala, Sweden; the NTA ligand is available from Quiagen GmbH) is first washed with eluent buffer containing 10 mM HEPES, 150 mM NaCl, 50 mM EDTA, 0.005% Surfactant P20, pH 7.4, followed by injection of 20 µl of 500 µM $NiCl_2$ at a flow rate of 20 µl/minute to saturate the NTA with $Ni^{2+}$. Subsequently a reporter polypeptide (0.01-100 mM in HEPES buffer) which has the poly histidine tag is applied to the sensor chip. This binds specifically to the Ni-NTA support. The sensorgram shows a rise indicative of mass increase and therefore establish a new baseline. Once this baseline is stable, cell lysate (solubilised in HEPES buffer), which is to be tested for phosphorylation activity is passed onto the sensor chip for periods of time ranging from 5 sec to 30 min. Again the sensorgram shows a rise indicative of mass increase and reaches a new steady state. The sample is removed by washing with HEPES buffer and the sensorgram signal falls to a baseline little different to the previous baseline.

Once this signal is stable, the partner peptide (0.01-100 mM in HEPES buffer) is then applied to the sensor chip. At this stage the sensorgram is recorded for the change in mass. When the immobilised peptide is phosphorylated, the peptide partner can bind to it, thus giving an increase in mass which is observed via a positive signal of the sensorgram. This is indicative of activity of a kinase which phosphorylates a site on the immobilised binding partner.

If the assays of the invention are to be performed using an alternative output method, such as FRET, a number of general techniques and test procedures are used. These are set out below, and find use in other embodiments of assays of this type.

Fluorescence measurements of protein modification *in vitro* in real time

Donor and acceptor fluorophore-labelled natural binding domains and the corresponding binding partners for any such natural molecules (molar equivalents of fluorophore-labelled polypeptide or molar excess of acceptor-labelled polypeptide) are first mixed (if the two are present on separate polypeptides). Samples are analyzed in a fluorimeter using excitation wavelengths relevant to the donor fluorescent label and emission wavelengths relevant to both the donor and acceptor labels. A ratio of emission from the acceptor over that from the donor following excitation at a single wavelength is used to determine the efficiency of fluorescence energy transfer between fluorophores, and hence their spatial proximity. Typically, measurements are performed at 0-37°C as a function of time following the addition of the modifying enzyme (and, optionally, a modulator or candidate modulator of function for that enzyme) to the system in 50 mM histidine pH 7.0, 120 mM KC1, 5 mM $MgSO_4$, 5 mM NaF, 0.05 mM EGTA and 0.2 mM ATP or a buffer optimal for the enzyme being used. The assay may be performed at a higher temperature if that temperature is compatible with the enzyme(s) under study.

Alternative cell-free assay system of the invention

A cell-free assay system must permit binding of a natural binding domain to its binding partner to occur in a modification-dependent manner. As indicated herein, such a system may comprise a low-ionic-strength buffer (e.g., physiological salt, such as simple saline or phosphate- and/or Tris-buffered saline), a cell culture medium, of which many are known in the art, or a whole or fractionated cell lysate. The components of an assay or post-translational modification of a polypeptide molecule may be added into a buffer, medium or lysate or may have been expressed in cells from which a lysate is derived. Alternatively, a cell-free transcription- and/or translation system may be used to deliver one or more of these components to the assay system. Nucleic acids of use in cell-free expression systems are as described for *in vivo* assays, below.

An assay of the invention may be performed in a standard *in vitro* transcription/translation system under conditions which permit expression of a recombinant or other gene. The TNT® T7 Quick Coupled Transcription/Translation System (Cat. # L1170; Promega) contains all reagents necessary for *in vitro* transcription/translation except the DNA of interest and the detection label, as discussed below, a natural binding domain and/or its binding partner may be encoded by expression of constructs in which their coding sequences are fused in-frame to those encoding fluorescent proteins. The TNT® Coupled Reticulocyte Lysate Systems (comprising a rabbit reticulocyte lysate) include: TNT® T3 Coupled Reticulocyte Lysate System (Cat # L4950; Promega); TNT® T7 Coupled Reticulocyte Lysate System (Cat. # L4610; Promega); TNT® SP6 Coupled Reticulocyte Lysate System (Cat. # L4600; Promega); TNT® T7/SP6 Coupled Reticulocyte Lysate System (Cat. # L5020; Promega); TNT® T7/T3 Coupled Reticulocyte Lysate System (Cat. # L5010; Promega).

An assay involving a cell lysate or a whole cell may be performed in a cell lysate or a whole cell, preferably eukaryotic in nature (such as yeast, fungi, insect, e.g., *Drosophila*), mouse, or human). An assay in which a cell lysate is used is performed in a standard *in vitro* system under conditions which permit gene expression. A rabbit reticulocyte lysate alone is also available from Promega, either nuclease-treated (Cat. # L4960) or untreated (Cat. # L4151).

*In vivo* assays of enzymatic activity

Methods are also provided for measuring enzyme activity via an effect on interactions between a binding domain and a binding partner thereof *in vivo*. For example, transgenic multicellular organisms may be produced that express in the same cell both the binding domain and binding partner. The binding of the binding domain and the binding partner to one another may be determined by means of a detectable label on at least one of the polypeptides, typically both. The physical characteristics of the labels alter in a measurable way depending on the molecular environment in which the polypeptides are found. Thus, in particular, the physical characteristics of the labels will alter depending on whether the binding domain and binding partner are bound to one another. Measurement of the physical characteristics of the labels will allow a determination of the extent of binding. The use of non-invasive measuring techniques will make it possible to follow the interactions over time in living cells, such as *in situ* in an intact multicellular organism and/or in a biological sample taken from a multicellular organism. The results obtained may be used to obtain physiologically relevant information such as kinetic and/or spatial information about the nature of the interaction between the binding domain and binding partner.

Clearly, in an *in vivo* situation, binding will be influenced by a number of factors such as polypeptide competitors and stimuli acting on signal transduction pathways leading to, for example, post-translational modifications, notably phosphorylation. Thus, an important application of the method of the invention is to study the effect on the binding of the binding domain to the binding partner of substances that stimulate cells within a transgenic organism, such as receptor agonists and antagonists.

For example, a compound may be known to have an inhibitory effect on the activity of a protein kinase *in vitro*. The protein kinase is typically known to phosphorylate and promote the binding of a binding domain to a binding partner. Thus, if the compound is administered to a transgenic animal that expresses the binding domain and binding partner, labelled with a detectable label, then the consequential effect on the association of the binding domain and binding partner may be assessed *in situ,* in particular in a range of different tissues to determine if the effect is confined to particular cell types.

The assay system may be used in an intact, living multicellular organism, such as an insect or a mammal. Methods of generating transgenic *Drosophila,* mice and other organisms, both transiently and stably, are well known in the art; detection of fluorescence resulting from the expression of Green Fluorescent Protein in live *Drosophila* is well known in the art. Where the expression of the nucleotide constructs is under the control of a inducer, sufficient time is allowed to pass after administration of the inducer molecule to allow for gene expression, for binding of a binding domain to its binding partner and for chromophore maturation, if necessary (e.g., Green Fluorescent Protein matures over a period of approximately 2 hours prior to fluorescence) before fluorescence or other emission from a detectable label is measured. A reaction component (particularly a candidate modulator of enzyme function) which is not administered as a nucleic acid molecule may be delivered by a method selected from those described below.

*In vivo* assays may be carried out *in situ* or using one or more cells removed from the transgenic organism. The one or more cells may be obtained from any subset of the tissues of the organism. Methods for visualising bioluminescence in living mammals are described in Contag *et al*., 1997, Photochemistry and Photobiology 66: 523-531 reviewed in Contag *et al*., 1998, Nature Medicine 4: 245-247 (see also references therein).

Alternatively, or in addition, *in vitro* assays of interactions between the binding domain and binding partner may be performed using extracts from a biological sample comprising one or more cells taken from a transgenic animal or body fluids, whether to test the activity of a recombinant protein or one which is found in nature. The use of a crude cell extract and/or body fluids enables rapid screening of many samples, which potentially finds special application in high-throughput screening methods, e.g., of candidate modulators of the binding domain-binding partner interaction. However, it is generally intended that *in vitro* assays are to be used as a precursor to, or for confirmation of, the *in vivo* assays.

As used herein, the term "biological sample" refers to a subset of the tissues of a biological organism, its cells or component parts (e.g., body fluids, including but not limited to blood, mucus, lymphatic fluid, synovial fluid, cerebrospinal fluid, saliva, amniotic fluid, amniotic cord blood, urine, vaginal fluid and semen). "Biological sample" and "biological specimen" further refer to a homogenate, lysate or extract prepared from a whole organism or a subset of its tissues, cells or component parts, or a fraction or portion thereof.

Sets of differentially-labelled binding domain and binding partner pairs are delivered to cells either by direct introduction of the polypeptides using techniques such as microinjection or by introducing nucleic acid constructs that express the polypeptides using standard techniques. The ratio of emission from the labelled molecule(s) may be measured for example using a photomultiplier tube focused on a single cell.

E. Protein modifications in assays of the invention

The invention provides methods for assaying the activity of enzymes which perform post-translational modification of proteins *in vivo*. Table 2 lists some non-limiting examples of post-translational modifications.

Table 2

| Modification | Protein Source | Consensus Sequence/ Sequence | Reference/ GenBank No. |
|---|---|---|---|
| | | Modified residues indicated in bold. Residues forming part of the recognition site are shown in italics. | |
| ADP-Ribosylation | B-50 | [1]ML**CC**MRRTKQV EKND DD | Coggins *et al*., 1993, J. Neurochem., 60: 368-71 |
| | γ subunit of cGMP phosphodiesterase | [30]FKQ**R**QT**R**QFK | X04270 |
| Ubiquitination | I B | [1]MFQAAERPQEW AMEG PRDGL**KK**ERLLD DRH | M69043 |

Table 2   (continued)

| Modification | Protein Source | Consensus Sequence/ Sequence | Reference/ GenBank No. |
|---|---|---|---|
| N-Myristoylation | Src | [1]**G**SSK*S*KPKD | Resh, 1994, Cell, 76: 411-413 |
| | Lyn | [1]**G***C*IK*S*KRKD | Resh, 1994, supra |
| | Yes | [1]**G***C*IK*S*KEDK | Resh, 1994, supra |
| | Fyn | [1]**G***C*VQ*C*KDKE | Resh, 1994, supra |
| | Gα | [1]**G***C*TL*S*AEDK | Resh, 1994, supra |
| Palmitylation | Lyn | [1]**GC**IK*S*KRKD | M64608 |
| | Fyn | [1]**GC**VQCKDKE | M14676 |
| | Gαi2 | [1]GCTLSAEDK | Milligan et al., 1995, Trends Biochem. Sci.. 20: 181-186 |
| N-Glycosylation | | -*NXS/T*- X can be any amino acid except P | Shakineshleman, 1996, Trends Glyco science and Glyco tech., 8: 115-130 |
| O-Glycosylation | p67[SRF] | [274]GTT**S**TIQTAP[3] [13]SAV**S**SADGTVL K[374]DSSTDLTQT**S** SSGTVTLP | J03161 |
| Sentrinization | RanGAP1 | | Johnson and Hochstrasser, 1997, Trends Cell. Biol., 7: 408-413 |
| | PML | | Kamitani et al., 1998, J. Biol. Chem., 273: 3117-3120 |

Phosphorylation - kinase and phosphatases

A particularly important post-translational modification for which a large number of enzymes and targets have been identified is phoshorylation and dephosphorylation. The art is replete with references to said enzymes, i.e. protein kinases and phosphatases, and their targets, including consensus phosphorylation motifs (such as -SQ- or -TQ- for the DNA dependent protein kinase (DNA-PK).

Some non-limiting examples of kinases and their sites for post-translational modification are presented in Table 3 (phosphorylation/dephosphorylation)

Table 3

| Kinase | Consensus Sequence | GenBank No./Reference |
|---|---|---|
| cAMP-dependent protein kinase | -[RR]XRRXS/T-([RR] not essential) | Cα subunit M34181 RIIβ subunit M31158 |
| Myosin Heavy Chain kinase | -KXX**S**X- | Trends in Biochem. Sci. (1990) <u>15</u> 342-346. |
| Myosin Heavy Chain kinase | -RX**T**- | M93393 |

Table 3   (continued)

| Kinase | Consensus Sequence | GenBank No./Reference |
|---|---|---|
| Calmodulin-Dependent protein kinase II | -RXX**S**X- | α chain J02942<br>β chain M16112<br>γ chain J05072<br>δ chain J04063 |
| cGMP-dependent protein kinase | -X**S**RX- | β isozyme Y07512 |
| Protein kinase C | -X**R**XXSXRX- | Trends in Biochem. Sci.(1990) 15 342-346. |
| S6 kinase II | -XRXX**S**X- | α2 isozyme L07599, L07601 |
| dsRNS kinase pp68 | -SEL**S**RR- | Trends in Biochem. Sci.(1990) 15 342-346. |
| Casein kinase I | -X**S**XXSX- | α isoform X80693 |
| Mammary gland casein kinase | -X**S**XEX- | Trends in Biochem. Sci.(1990) 15 342-346. |
| Glycogen synthase kinase 3 | -XSXXX**S**X | α isoform L40027 |
| X signifies any amino acid. Consensus sequences are taken from Trends Biochem. Sci. (1990) 15: 342-346. | | |

Further examples of protein kinases identified to date include the protein tyrosine kinase subfamily (such as PDGF receptors, EGF receptors, src family kinases (see Brown and Cooper, 1996, Biochimica and Biophysica Acta 1287: 121-149 for a review), the JAK kinase family (such as JAK1, JAK2 and tyk2), Erb B2, Bcr-Abl, Alk, Trk, Res/Sky - for a detailed review see Al-Obeidi *et al.*, 1998, Biopolymers (Peptide Science), Vol 47: 197-223), the MAP kinase pathway subfamily (such as the MAP family, the ERK family, the MEK family, the MEKK family, RAF-1 and JNK), the cyclin-dependent kinase subfamily (such as p34cdc2 and cdk2 - see Nigg, 1995, Bioessays 17: 471-480 for a review), Wee1/Myt1, polo-like kinases (such as plk1, Plx1, POLO, Snk, Fnk/Prk Sak-a, Sak-b - see Lane and Nigg, 1997, Trends in Cell Biol. 7: 63-68), the receptor serine kinase subfamily, protein kinase C (PK-C), cyclic-AMP dependent kinase (PK-A), cyclic-GMP dependent kinase (PKG), Ca2+/calmodulin dependent kinases (such as CaM kinase I, II and IV), DNA dependent protein kinase,), phosphoinositide 3-kinases (P13K), PDK-1, the p21-activated protein kinase family (PAKs), such as Pak1, Pak2 and Pak3- see Sells and Chernoff, 1997, Trends in Cell Biol. 7: 162-167), p70 S6 kinase, IkB kinase, casein kinase II, glycogen-synthase kinases (GSK3).

A discussion of particular kinase pathways involved in signal transduction is given in chapter 35 of Lewin, 1997, Gene VI, Oxford University Press.

Details of recognition and binding domains for a variety of kinases are given in Kuriyan and Cowburn, 1997, Annu. Rev. Biophys. Biomol. Struc. 26:259-288.

Some specific examples of kinases whose activity may be studied using the methods of the invention include the src family tyrosine kinases Lck and Fyn, that phosphorylate the TCR ζ chain, and are known to be involved in signal transduction associated with T cell receptor stimulation. The TCR ζ chain comprises specific tyrosine residues present in immunoreceptor tyrosine-based activation motifs (ITAMs) that are phosphorylatd by Lck and Fyn (Kuriyan and Cowbum, 1997, *ibid*.). The SH2 domain of another tyrosine kinase, ZAP70 binds to phosphorylated TCR ζ. Thus TCR ζ ITAM and ZAP70 SH2 represent binding domains and binding partners that may be of interest in studying the activity of the kinases Lck and Fyn (see Elder et al., 1994, Science 264: 1596-1599 and Chan et al., 1994, Science 264: 1599-1601.

Another example is the IgE receptor γ subunit and the SH2 domain of Syk that may be used to study the activity of the Lyn kinase.

Examples of phosphatases identified to date fall into three main families (for review see Barford et al., 1998, Annu. Rev. Biophys. Biomol. Struc. 27: 133-164). The PPP family includes the following catalytic subunits: PP1c, PP2Ac, PP2B, PPP1, PPP2A and PPP5 and the following regulatory subunits: NIPP-1, RIPP-1, p53BP2, $\gamma_1$34.5, PR65, PR55, PR72, PTPA, SV40 small T antigen, PPY, PP4, PP6 and PP5.

The PPM family includes pyruvate dehydrogenase phosphatase and *Arabidopsis* ABI1.

The protein tyrosine phosphatase family includes PTP1B, SHP-1, SHP-2 (cytosolic non-receptor forms), CD45 (see Thomas and Brown, 1999, Trends in Immunol, 20: 406 and Ashwell and D'Oro, 1999, Trends in Immunol, 20: 412 for further details), RPTP (receptor-like, transmembrane forms) and cdc25, kinase-associated phosphatase and MAP kinase phosphatase-1 (dual-specificity phosphatases). PTP1B is known to associate with the insulin receptor *in vivo* (Bandyopadhyay *et al.*, 1997, J. Biol. Chem. 272: 1639-1645).

A simple FRET assay based upon these modifications to site for post-translational modification present on a natural binding domain may be performed as presented below.

$$\text{(F1-NBD)(F2-partner)} + \text{substrate} \rightarrow \text{F1-M-NBD} + \text{F2-partner} + \text{byproduct}$$
$$\text{(FRET)} \qquad\qquad \text{(No FRET)}$$

where NBD = natural binding domain; M = modification; F1 = donor fluorophore and F2 = acceptor fluorophore

Alternatively, a FRET-based assay may follow a format such as:

$$\text{F1-NBD} + \text{F2-partner} + \text{substrate} \rightarrow \text{(F1-M-NBD)(F2-partner)} + \text{byproduct}$$
$$\text{(No FRET)} \qquad\qquad \text{(FRET)}$$

Table 4 provides a non-limiting list of enzymes that are representative of some of the classes of modifying enzymes discussed herein as being amenable to assay according to the invention.

Table 4

| Modification | Enzyme | Specific Action | GenBank No./ Reference |
|---|---|---|---|
| Mono-ADP-Ribosylation | NAD:Arginine ADP-ribosyl transferase | | Zolkiewska *et al.,* 1992, PNAS B2: 11352-6 |
| Poly- ADP-Ribosylation | *Drosophila* PARP | | D13806, D 13807, D13808 |
| Ubiquitination | E1 E2(UBC8) E3(RSP5) | Ubiquitination of large subunit of RNA pol II (Rpb 1) (NB, E2 and E3 confer substrate specificity on the ubiquitination) | X55386, X56507, M65083, U18916, L11119, L11120, U00092, U75972 |
| N-Myristoylation | Glycylpeptide-N-tetra decanoyltransferase (peptide N-myristoyl transferase) | | M86707 |
| N-Glycosylation | UDP-N-acetylglucosaminedolichyl-phosphate N-acetylglucosamine phosphotransferase | Initial step in synthesis of dolichol-P-P-oligosacharides | X65603, S41875 |
| O-Glycosylation | O-GlcNAc transferase | | Kreppel *et al.*, 1997, J. Biol. Chem., 272: 9308-9315 |
| Sentrinization | Ubc9 | | Gong *et al.*, 1997, J. Biol. Chem., 272, 28198-28201 |
| (de)phosphorylation | Kinase/phosphatase | | See above |

The several types of post-translational modification presented above will be discussed in some detail below, along with assays to test the enzymes that perform such modifications using natural binding domains and binding partners therefor.

ADP-ribosylation

Mono-ADP-ribosylation is a post-translational modification of proteins which is currently thought to play a fundamental role in cellular signalling. A number of mono-ADP-ribosyl-transferases have been identified, including endogenous enzymes from both bacterial and eukaryotic sources and bacterial toxins. A mono-ADP-ribosylating enzyme, using as substrates the protein to be modified and nicotinamide adenine dinucleotide ($NAD^+$), is NAD:Arginine ADP ribosyltransferase (Zolkiewska *et al.*, 1992, Proc. Natl. Acad. Sci. U.S.A., 89: 11352-11336). The reactions catalyzed by bacterial toxins such as cholera and pertussis toxin are well understood; the activities of these toxins result in the permanent modification of heterotrimeric G proteins. Endogenous transferases are also thought to modify G proteins and therefore to play a role in signal transduction in the cell (de Murcia *et al.*, 1995, Trends Cell Biol., 5: 78-81). The extent of the effects that ADP-ribosylation can mediate in the cell is illustrated by the example of brefeldin A, a fungal toxin metabolite of palmitic acid. This toxin induces the mono-ADP-ribosylation of BARS-50 (a G protein involved in membrane transport) and glyceraldehyde-3-phosphate dehydrogenase. The cellular effects of brefeldin A include the blocking of constitutive protein secretion and the extensive disruption of the Golgi apparatus. Inhibitors of the brefeldin A mono-ADP-ribosyl-transferase reaction have been shown to antagonise the disassembly of the Golgi apparatus induced by the toxin (Weigert *et al.*, 1997, J. Biol. Chem., 272: 14200-14207). A number of amino acid residues within proteins have been shown to function as ADP-ribose acceptors. Bacterial transferases have been identified which modify arginine, asparagine, cysteine and diphthamide residues in target proteins. Endogenous eukaryotic transferases are known which also modify these amino acids, in addition there is evidence that serine, threonine, tyrosine, hydroxyproline and histidine residues may act as ADP-ribose acceptors but the relevant transferases have not yet been identified (Cervantes-Laurean *et al.*, 1997, Methods Enzymol., 280: 275-287 and references therein).

Poly-ADP-ribosylation is thought to play an important role in events such as DNA repair, replication, recombination and packaging and also in chromosome decondensation. The enzyme responsible for the poly-ADP-ribosylation of proteins involved in these processes is poly (ADP-ribose) polymerase (PARP; for *Drosophila melanogaster* PARP, see Genbank Accession Nos. D13806, D13807 and D13808). The discovery of a leucine zipper in the self-poly (ADP-ribosyl)ation domain of the mammalian PARP (Uchida *et al.*, 1993, Proc. Natl. Acad. Sci. U.S.A., 90: 3481-3485) suggested that this region may be important for the dimerization of PARP and also its interaction with other proteins (Mendoza-Alvarez *et al.*, 1993, J. Biol. Chem., 268: 22575-22580).

Specific examples of ADP-ribosylation sites are those found at $Cys_3$ and $Cys_4$ (underlined) of the B-50 protein (Coggins *et al.*, 1993, J. Neurochem., 60: 368-371; SwissProt Accession No. P06836): ML<u>CC</u>MRRTKQVEKNDDD and Pγ (the γ subunit of cylic CMP phophodiesterase; Bondarenko *et al.*, 1997, J. Biol. Chem., 272: 15856-15864; Genbank Accession No. X04270): FKQ<u>R</u>QT<u>R</u>QFK.

Glycosylation

N-linked glycosylation is a post-translational modification of proteins which occurs in the endoplasmic reticulum and golgi apparatus and is utilized with some proteins *en route* for secretion or destined for expression on the cell surface or in another organelle. The carbohydrate moiety is attached to Asn residues in the non-cytoplasmic domains of the target proteins, and the consensus sequence (Shakineshleman, 1996, Trends Glycosci. Glycotech., 8: 115-130) for a glycosylation site is NxS/T, where x cannot be proline or aspartic acid.

N-linked sugars have a common five-residue core consisting of two GlcNAc residues and three mannose residues due to the biosynthetic pathway. This core is modified by a variety of Golgi enzymes to give three general classes of carbohydrate known as oligomannosyl, hybrid and lactosamine-containing or complex structures (Zubay, 1998, Biochemistry, Wm. C. Brown Publishers). An enzyme known to mediate N-glycosylation at the initial step of synthesis of dolichyl-P-P-oligosaccharides is UDP-N-Acetylglucosamine-Dolichyl-phosphate-N-acetylsglucosamine phosphotransferase (for mouse, Genbank Accession Nos. X65603 and S41875).

Oxygen-linked glycosylation also occurs in nature with the attachment of various sugar moieties to Ser or Thr residues (Hansen *et al.*, 1995, Biochem. J., 308: 801-813). Complex O-linked glycosylation can be broken into at least six classes - mucin type, ser- 1-GlcNAc; proteoglycan type, ser-Gal-Gal-Xyl core; collagen type, hydroxylys-Gal-Glc; clotting factor type, ser-Xyl-Glc or ser-Xyl-Xyl-Glc core; fungal type, ser-Man; plant type, hydroxypro-Ara or ser-Gal (where GlcNAc = N-acetylglucosamine, Gal = galactose, Xyl = Xylose; Glc = glucose, Man = mannose and Ara = arabinose; Hansen *et al.*, 1995, supra). Intracellular proteins are among the targets for O-glycosylation through the dynamic attachment and removal of O-N-Acetyl-D-glucosamine (O-GlcNAc; reviewed by Hart, 1997, Ann. Rev. Biochem., 66: 315-335). Proteins known to be O-glycosylated include cytoskeletal proteins, transcription factors, the nuclear pore protein complex, and tumor-suppressor proteins (Hart, 1997, supra). Frequently these proteins are also phosphoproteins, and there is a suggestion that O-GlcNAc and phosphorylation of a protein play reciprocal roles. Furthermore, it has been proposed that the glycosylation of an individual protein regulates protein:protein interactions in which it is involved.

Specific sites for the addition of O-GlcNAc are found, for example, at $Ser_{277}$, $Ser_{316}$ and $Ser_{383}$ of p67$^{SRF}$ (Reason *et al.*, 1992, J. Biol. Chem., 267: 16911-16921; Genbank Accession No. J03161). The recognition sequences encompassing these residues are shown below:

$^{274}$GTTSTIQTAP $^{313}$SAVSSADGTVLK $^{374}$DSSTDLTQTSSSGTVTLP

The identity of sites of O-GlcNAc is additionally known for a small number of proteins including c-myc ($Thr_{58}$, also a phosphorylation site; Chou *et al.*, 1995, J. Biol. Chem., 270: 18961-18965), the nucleopore protein p62 (see Reason *et al.*, 1992, supra): MAGGPADTSDPL and band 4.1 of the erythrocyte (see Reason *et al.*, 1992, supra): AQTITSETPSSTT.

The site at which modification occurs is, in each case, underlined. The reaction is mediated by O-GlcNAc transferase (Kreppel *et al.*, 1997, J. Biol. Chem., 272: 9308-9315).

Prenylation (fatty acylation)

The post-translational modification of proteins with fatty acids includes the attachment of myristic acid to the primary amino group of an N-terminal glycine residue (Johnson *et al.*, 1994, Ann. Rev. Biochem., 63: 869-914) and the attachment of palmitic acid to cysteine residues (Milligan *et al.*, 1995, Trends Biochem. Sci., 20: 181-186).

Fatty acylation of proteins is a dynamic post-translational modification which is critical for the biological activity of many proteins, as well as their interactions with other proteins and with membranes. Thus, for a large number of proteins, the location of the protein within a cell can be controlled by its state of prenylation (fatty acid modification) as can its ability to interact with effector enzymes (ras and MAP kinase, Itoh *et al.*, 1993, J. Biol. Chem., 268: 3025-; ras and adenylate cyclase in yeast; Horiuchi *et al.*, 1992, Mol. Cell. Biol., 12: 4515) or with regulatory proteins (Shirataki *et al.*, 1991, J. Biol. Chem., 266: 20672-20677). The prenylation status of ras is important for its oncogenic properties (Cox, 1995, Methods Enzymol., 250: 105-121) thus interference with the prenylation status of ras is considered a valuable anti-cancer strategy (Hancock, 1993, Curr. Biol., 3: 770).

Sentrinization

Sentrin is a novel 101-amino acid protein which has 18% identity and 48% similarity with human ubiquitin (Okura *et al.*, 1996, J. Immunol., 157: 4277-428 1). This protein is known by a number of other names including SUMO-1, UBL1, PIC1, GMP1 and SMT3C and is one of a number of ubiquitin-like proteins that have recently been identified. Sentrin is expressed in all tissues (as shown by Northern blot analysis), but mRNA levels are higher in the heart, skeletal muscle, testis, ovary and thymus.

The sentrinization of proteins is thought to involve the Ubiquitin-conjugating enzyme Ubc9 (Gong *et al.*, 1997, J. Biol. Chem., 272: 28198-28201). The interaction between these two proteins in the yeast two-hybrid screen is very specific, suggesting that this is a biologically relevant phenomenon. The interaction is dependent upon the presence of the conserved C-terminal Gly-Gly residues present in sentrin (Gong *et al.*, 1997, supra). The conjugation of sentrin to other proteins *via* Gly97 requires the cleavage of the C-terminal four amino acids of the protein, His-Ser-Thr-Val.

One important protein shown to be modified by the addition of sentrin is the Ran-specific GTPase-activating protein, RanGAP1, which is involved in nuclear import of proteins bearing nuclear-localization signals (Johnson and Hochstrasser, 1997, Trends Cell Biol., 7: 408-413). Conjugation of RanGAP1 by sentrin is essential both for the targeting of RanGAP1 to its binding partner on the nuclear pore complex (NPC) and for the nuclear import of proteins. Sentrin itself does not bind with high affinity to the NPC and it is, therefore, likely that it either provokes a conformational change in RanGAP1 that exposes a binding site or, alternatively, that the binding site is formed using both sentrin and RanGAP1 sequences. There is evidence to suggest that the conjugation of sentrin to RanGAP1 is necessary for the formation of other sentrinized proteins (Kamitani *et al.*, 1997, J. Biol. Chem., 272: 14001-14004) and that the majority of these sentrinized proteins are found in the nucleus.

Sentrin has been shown in yeast two-hybrid screens to interact with a number of other proteins, including the death domains of Fas/APO1 and the TNF receptors, PML, RAD51 and RAD52 (Johnson and Hochstrasser, 1997, supra). These interactions implicate sentrin in a number of important processes. Fas/APO1 and TNF receptors are involved in transducing the apoptosis signal via their death domains. Ligation of Fas on the cell surface results in the formation of a complex *via* death domains and death-effector domains, triggering the induction of apoptosis. The overexpression of sentrin protects cells from both anti-Fas/APO and TNF-induced cell death (Okura *et al.*, 1996, supra). It is not clear whether this protection is achieved simply by preventing the binding of other proteins to these death domains or whether a more complex process is involved, possibly one involving the ubiquitin pathway.

The interaction of sentrin with PML (a RING finger protein) is important, as it points to a disease state in which this protein may play a role. In normal myeloid cells, PML is found in a nuclear multiprotein complex known as a nuclear body. These nuclear bodies are disrupted in acute promyelocytic leukaemia, where a chromosomal translocation generates a fusion between regions of the retinoic acid receptor and PML. This disruption can be reversed by treatment with retinoic acid. It has been shown that PML is covalently modified at multiple sites by members of the sentrin family of proteins (but not by ubiquitin or NEDD8). Two forms of the aberrant fusion protein have been identified, neither of which is modified by sentrin. It is, therefore, thought that differential sentrinization of the normal and abberant forms of PML may be important in the processes underlying acute promyelocytic leukaemia and may help in the understanding of the biological role of the PML protein (Kamitani *et al.*, 1998, J. Biol. Chem., 273: 3117-3120).

Proteases

As noted above, susceptibility to modification includes the possibility that the polypeptide may be subjected to proteolytic degradation under the appropriate conditions, which in a preferred embodiment means that the polypeptide is cleaved by a protease at a recognition site for the protease enzyme. Alternatively, however, the polypeptide may be susceptible to digestion by an exoprotease, from the N or C terminus.

Where engineered polypeptides are used, they should be constructed such that the protease cleavable site is positioned such that cleavage thereof disrupts binding of the polypeptide in the context of the multimer. Thus, polypeptides which have been subjected to protease cleavage should dissociate from the multimer. Preferably, the protease does not cleave the polypeptide in such a manner that the label becomes detached therefrom without the binding abilities thereof being disrupted. Location of the protease cleavable site may be determined empirically. As a guide, however, the site should be placed within or proximal to the binding domain which is responsible for the multimerisation of the polypeptide.

In the case of coiled coil binding domains, Lumb *et al* (Subdomain folding of the coiled coil leucine zipper from the bZIP transcriptional activator GCN4, Lumb, K.J., Carr, C.M. & Kim, P.S., Biochemistry (1994) 33 7361-7367) teach that the loss of ten residues from the N-terminus or seven from the N- and six from the C-terminus is sufficient to destabilise the coiled coil peptide sequence known as GCN4-p1 (Evidence that the leucine zipper is a coiled coil, O'Shea, E.K., Rutkiowski, R. & Kim, P.S. (1989) Science *243* 538-542). Su *et al* provide data indicating that there is a sharp decrease in the stability of a designed coiled coil with a decrease in chain length from 23 to 19 residues (Su, J. Y., Hodges, R.S. & Kay, C.M., Biochemistry (1994) 33 15501-15510). Accordingly, cleavage sites may be positioned such that the coiled coil is disrupted to an extent that it is no longer capable of directing multimerisation.

A number of proteases and their cleavage sites are known in the art, and set forth in Table 5.

Table 5

| Protease | Cut Site(s) | Possible/Proven Role |
|---|---|---|
| Aminopeptidase M | Hydrolysis from free N-terminus | digestion |
| Carboxypeptidase Y | Hydrolysis from C-terminus | digestion |
| Caspase 1,4,5 | W/LEHD-X[#] | mediator of apoptosis |
| Caspase 2,3,7 | DEXD-X[#] | mediator of apoptosis |
| Caspase 6,8,9 | L/VEXD-X[#] | mediator of apoptosis |
| Chymotrypsin | Y-X, F-X, T-X, (L-X, M-X, A-X, E-X) | digestion |
| Factor Xa | IEGR-X | blood clotting cascade |
| Pepsin | F-Z, M-Z, L-Z, W-Z (where Z is a hydrophobic residue) but will cleave others | digestion |
| TEV | E(N)XYXQ-S/G[~] | polyprotein processing / as a reagent |
| Thrombin | R-X | blood clotting cascade |
| Trypsin | R-X, K-X | digestion |

[#] Ideal cut sites identified by Thornberry *et al* in *A combinatorial approach defines specificities of members of the caspase family and granzyme B*, Journal of Biological Chemistry 272 17907-17911.

[~] *Release of proteins and peptides from fusion proteins using a recombinant plant virus proteinase,* Parks, T.D., Keuther, K.K., Howard, E.D., Johnston, S.A. & Dougherty, W.G., Analytical Biochemistry (1994) *216* 413-417; Life Technologies Ltd.

The foregoing, or other, sites may be engineered into or close to the binding domains of polypeptides used in the methods of the invention.

In a preferred aspect of the present invention, it is desirable to engineer specificity into the polypeptide, such that it is digested only by the desired protease and only at the intended protease cleavable site. This may be achieved, for example, by the use of D-isomers of amino acids in the construction of the polypeptide. D-amino acids are resistant to protease digestion, and a polypeptide constructed of D-amino acids will withstand proteolytic attack. Moreover, use of D-amino acids does not interfere with the protein-protein interactions involved in multimerisation, such as the interaction of protein binding domains, especially coiled-coil domains, provided that D-amino acids are employed in both members of a binding pair.

In order to allow digestion by the intended protease enzyme, the D-amino acid constructions of the polypeptides contain one or more parts constructed of L-amino acids, or otherwise rendered susceptible to proteolytic digestion. For example, coiled coils constructed of D-amino acids preferably comprise inserts, constructed wholly or partly of L-amino acids, which contain the protease cleavage site. The L-amino acid insert may be of any size, and may be positioned between coiled coil repeats, or between residues of the coiled coil. Preferred are insertions at positions b-c, e-f and f-g. The insert is covalently attached to the coiled coil, through a covalent linkage to the backbone or through a sidechain.

The insert may comprise only a cleavage site, or an entire polypeptide. Functionally, the insert is sufficiently flexible to permit the coiled coil to bind to its target efficiently when the insert is intact. For example, the insert may comprise a flexible linker, such as a gly-gly linker. Molecules comprising D-amino acids are advantageously employed in *in vitro* assays.

Inserts as described above may be employed in D-amino acid coiled coils, in conventional L-amino acid coiled coils, or in coiled coils which are partially D and partially L in construction. For example, a coiled coil may be constructed such that it consist of L-amino acids on one side of the insert, and D-amino acids on the other side thereof.

Candidate modulators of protein-modifying enzymes to be screened according to the invention

Whether *in vitro* or in an *in vivo* system the invention encompasses methods by which to screen compositions which may enhance, inhibit or not affect (e.g., in a cross-screening procedure in which the goal is to determine whether an agent intended for one purpose additionally affects general cellular functions, of which protein modification is an example) the activity of a protein-modifying enzyme.

Candidate modulator compounds from large libraries of synthetic or natural compounds can be screened. Numerous means are currently used for random and directed synthesis of saccharide, peptide, and nucleic acid based compounds. Synthetic compound libraries are commercially available from a number of companies including Maybridge Chemical Co. (Trevillet, Cornwall, UK), Comgenex (Princeton, NJ), Brandon Associates (Merrimack, NH), and Microsource (New Milford, CT). A rare chemical library is available from Aldrich (Milwaukee, WI). Combinatorial libraries are available and can be prepared. Alternatively, libraries of natural compounds in the form of bacterial, fungal, plant and animal extracts are available from e.g., Pan Laboratories (Bothell, WA) or MycoSearch (NC), or are readily produceable by methods well known in the art. Additionally, natural and synthetically produced libraries and compounds are readily modified through conventional chemical, physical, and biochemical means.

Useful compounds may be found within numerous chemical classes, though typically they are organic compounds, including small organic compounds. Small organic compounds have a molecular weight of more than 50 yet less than about 2,500 daltons, preferably less than about 750, more preferably less than about 500 daltons. Exemplary classes include heterocycles, peptides, saccharides, steroids, and the like. The compounds may be modified to enhance efficacy, stability, pharmaceutical compatibility, and the like. Structural identification of an agent may be used to identify, generate, or screen additional agents. For example, where peptide agents are identified, they may be modified in a variety of ways to enhance their stability, such as using an unnatural amino acid, such as a D-amino acid, particularly D-alanine, by functionalizing the amino or carboxylic terminus, e.g. for the amino group, acylation or alkylation, and for the carboxyl group, esterification or amidification, or the like.

Candidate modulators which may be screened according to the methods of the invention include receptors, enzymes, ligands, regulatory factors, and structural proteins. Candidate modulators also include nuclear proteins, cytoplasmic proteins, mitochondrial proteins, secreted proteins, plasmalemma-associated proteins, serum proteins, viral antigens, bacterial antigens, protozoal antigens and parasitic antigens. Candidate modulators additionally comprise proteins, lipoproteins, glycoproteins, phosphoproteins and nucleic acids (e.g., RNAs such as ribozymes or antisense nucleic acids). Proteins or polypeptides which can be screened using the methods of the present invention include hormones, growth factors, neurotransmitters, enzymes, clotting factors, apolipoproteins, receptors, drugs, oncogenes, tumor antigens, tumor suppressors, structural proteins, viral antigens, parasitic antigens, bacterial antigens and antibodies (see below).

Candidate modulators which may be screened according to the invention also include substances for which a test cell or organism might be deficient or that might be clinically effective in higher-than-normal concentration as well as those that are designed to eliminate the translation of unwanted proteins. Nucleic acids of use not only may encode the candidate modulators described above, but may eliminate or encode products which eliminate deleterious proteins. Such nucleic acid sequences are antisense RNA and ribozymes, as well as DNA expression constructs that encode them. Note that antisense RNA molecules, ribozymes or genes encoding them may be administered to a test cell or organism by a method of nucleic acid delivery that is known in the art, as described below. Inactivating nucleic acid sequences may encode a ribozyme or antisense RNA specific for the target mRNA. Ribozymes of the hammerhead class are the smallest known, and lend themselves both to *in vitro* production and delivery to cells.

Determination of activity of candidate modulator of a protein-protein interaction or protein-modifying enzyme

A candidate modulator of the interaction between a binding domain and a binding partner assayed according to the invention as described herein, is determined to be effective if its use results in a difference of about 10% or greater relative to controls in which it is not present (see below) in FRET or other signal emanating from a detectable label of use in the invention resulting from the association of a natural binding domain with its binding partner.

The level of activity of a candidate modulator may be quantified using any acceptable limits, for example, via the following formula:

$$\text{Percent Modulation} = \frac{(\text{Index}_{Control} - \text{Index}_{Sample})}{(\text{Index}_{Control})} \times 100$$

where $\text{Index}_{Control}$ is the quantitative result (e.g., amount of or rate of change in fluorescence at a given frequency, rate of molecular rotation, FRET, rate of change in FRET or other index of modification, including, but not limited to, enzyme inhibition or activation) obtained in assays that lack the candidate modulator (in other words, untreated controls), and $\text{Index}_{Sample}$ represents the result of the same measurement in assays containing the candidate modulator. As described herein, control measurements are made with a differentially-labelled natural binding domain and its corresponding partner only and with these molecules plus a protein-modifying enzyme which recognizes a natural site for post-translational protein modification present on the binding domain and, optionally, on the binding partner.

Such a calculation is used in either *in vitro* or *in vivo* assays performed according to the invention.

According to the invention, assays of the activity of protein-modifying enzymes may be performed using crude cellular extracts, whether to test the activity of a recombinant protein or one which is found in nature, such as in a biological sample obtained from a test cell line or animal or from a clinical patient. In the former case, use of a crude cell extract enables rapid screening of many samples, which potentially finds special application in high-throughput screening methods, e.g., of candidate modulators of protein-modifying enzyme activity. In the latter case, use of a crude extract with the labelled reporter polypeptide comprising a natural binding domain and, for at least one member of a pair of binding partners, a natural site for post-translational protein modification facilitates easy and rapid assessment of the activity of an enzyme of interest in a diagnostic procedure, e.g., one which is directed at determining whether a protein-modifying enzyme is active at a physiologically-appropriate level, or in a procedure designed to assess the efficacy of a therapy aimed at modulating the activity of a particular enzyme.

The present invention will now be described with reference to the following examples that are intended to be illustrative only and non-limiting. The Examples refer to Figures.

Description of the figures

Figure 1 - Homodimer based PKA FRET assay

Figure 2 - Heterodimer FRET signal

Figure 3 - schematic representation of heterodimer FRET assays where either one or both peptides have modification sites.

Figure 4 - PKA FRET assay using coiled-coil heterodimer substrates

Figure 5 - Heterodimer based fluorescence polarisation assay for PKA

Figure 6 - Comparison of PKA assay initial reaction rates using FRET or FP readouts

Figure 7 - Inhibition of PKA FRET Assays for PKA Inhibitor profile, measured using FRET readout

Figure 8 - FRET based assay of CaMK-11

Figure 9 - Inhibition of CaMK II by $CA^{2+}$/CaMK Inhibitor, 281-301 inhibitor using FRET based assay

Figure 10 - FP assay of CaMK II

Figure 11 - Simultaneous PKA and TEV assay
Figure 12 - Simultaneous assay of CaMK and PKA with FP readouts
Figure 13 - Simultaneous assay of Chk1 and PKA. Enzymes added after 10 minutes equilibration

## EXAMPLES

**Reference Example 1 - Assays for kinase activity using FRET and FP**

Materials and methods

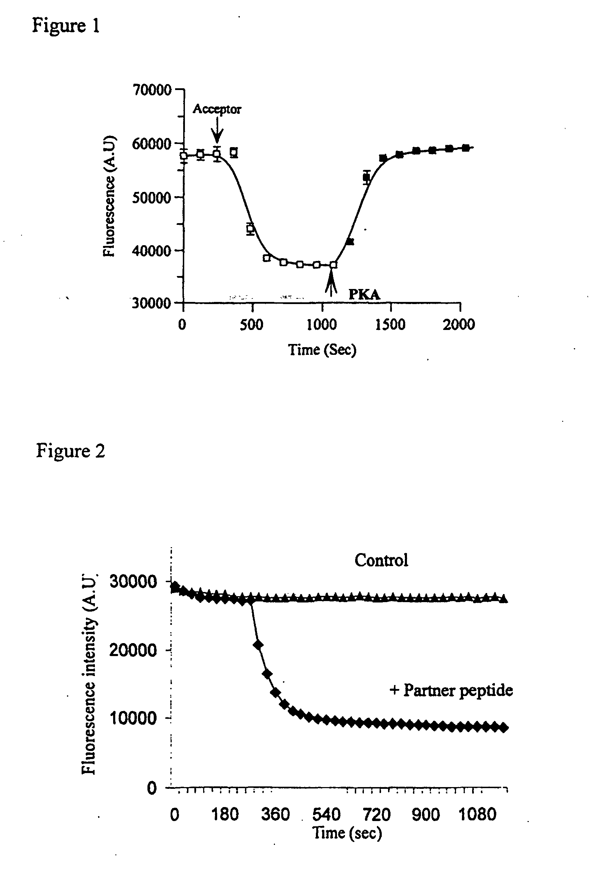

**Homodimer based kinase assay (Figure 1)**

Coiled-coil reporter peptides containing phosphorylation sites for PKA were labelled with fluorescein (F) or tetramethyl rhodamine (R). Labelled peptides (3.2 µM total peptide, 6:1 ratio R:F label) were incubated at 30°C in 50 mM Histidine pH 7.0, 5 mM MgSO$_4$, 120 mM KCl, 5 mM NaF, 0.2 mg/ml BSA, and 1 mM ATP in a total volume 100µl in a microtitre plate. Phosphorylation was initiated by the addition of PKA (3 pmoles), and monitored during the time course by following the fluorescence emission of fluorescein (at 520 nm, excitation 485 nm).

**Peptides used**          RMKQLEDQV**RRLRRKS**YHLENEVACLKKLVQELAAK
                          RMKQLEDQV**RRLRRKS**YHLENEVACLKKLVQELAAK

**Heterodimer based kinase assays**

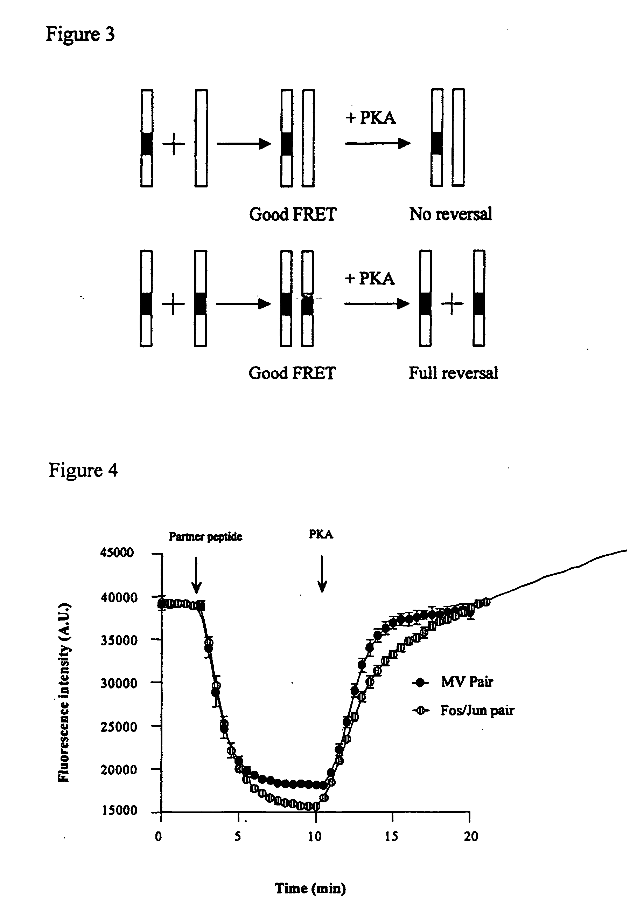

*(A) Heterodimer PKA FRET assay (Figures 4, 6, 7)*

Coiled-coil reporter peptides containing phosphorylation sites for PKA were labelled with fluorescein (F) or tetramethyl rhodamine (R). Labelled peptides (3.2 µM total peptide, 1:1 ratio R:F label) were incubated at 30°C in 50 mM Histidine pH 7.0, 5 mM MgSO$_4$, 120 mM KCl, 5 mM NaF, 0.2 mg/ml BSA, and I mM ATP in a total volume of 1.00 µl in a microtitre plate. Phosphorylation was initiated by the addition of PKA (3 pmoles), and monitored during the time course by following the fluorescence emission of fluorescein (at 520 nm, excitation 485 nm).

**Peptides used**       RMRCLEDEV**RRLRRKS**YHLANTVARLRQRVQELKAR
**Fos/Jun pair**        LMCQLQDEV**RRLRRKS**YHLQNEVARLLREVQELEAE


                        EREIKALEREI**RRLRRAS**QALEREIAQLERÈ
**Molecular velcro pair**  LRQRIACLRQRI**RRLRRAS**QALRQRIAQLKQR

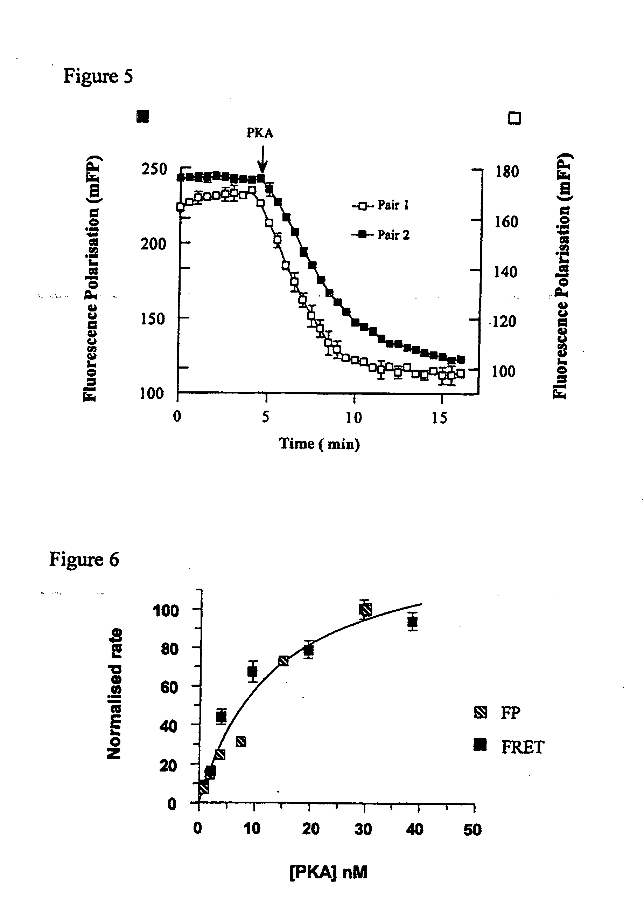

*(B) Heterodimer PKA FP assay (Figures 5, 6)*

Coiled-coil reporter peptides containing phosphorylation sites for PKA were labelled with fluorescein (F) or biotin (B). Labelled peptides (3.2 µM total peptide, 1:1 ratio B:F label) were incubated at 30°C in 50 mM Histidine pH 7.0, 5 mM MgSO$_4$, 120 mM KCl, 5 mM NaF, 0.2 mg/ml BSA, and 1mM ATP in a total volume of 100 µl in a microtitre plate. Streptavidin (0.1U) was added to each well and the samples were incubated at 30°C for 30 min. Phosphorylation was initiated by the addition of PKA (3 pmoles), and monitored during the time course by measuring the fluorescence polarisation (excitation 485nm, emission 520nm).

| Peptides used | RMRCLEDEV**RRLRRKS**YHLANTVARLRQRVQELKAR |
|---|---|
| (PKA Fos/Jun) | LMCQLQDEV**RRLRRKS**YHLQNEVARLLREVQELEAE |

| | EREIKALEREI**RRLRRAS**QALEREIAQLERE |
|---|---|
| (PKA Mol. Velcro) | LRQRIQCLRYRI**RRLRRAS**QALRQRIAQLKQR |

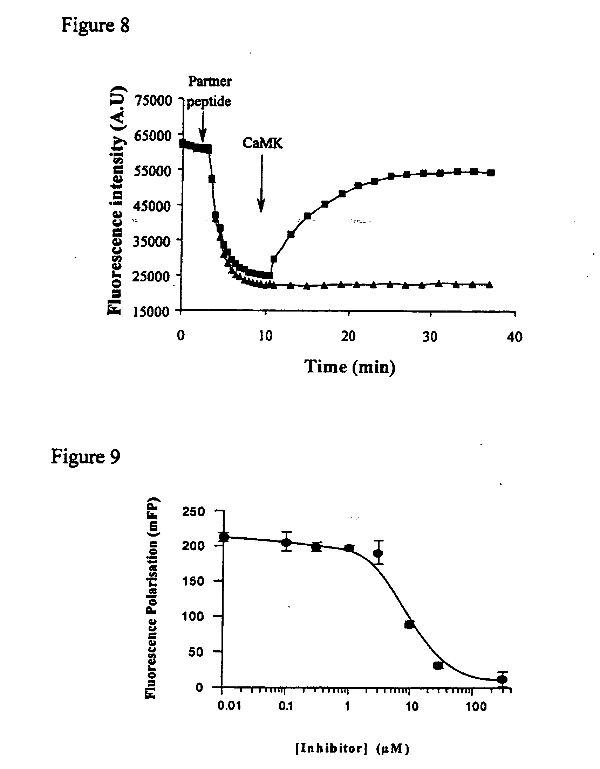

*(C) CaMK FRET assay - Heterodimer (Figures 8, 9)*

Coiled-coil reporter peptides containing phosphorylation sites for calmodulin-dependent kinase II were labelled with fluorescein (F) or tetramethyl rhodamine (R). Labelled peptides (5 to 20 µM total peptide, 4:1 ratio R:F label) were incubated at 30°C in 50 mM MOPS pH 7.2, 5 mM $MgCl_2$, 0.4 mM DTT, 0.1 mM $CaCl_2$, 0.1 mg/ml BSA, 0.5 µM calmodulin, and 1 mM ATP in a total volume of 100 µl in a microtitre plate. Phosphorylation was initiated by the addition of calmodulin dependent kinase II (1 pmole), and monitored during the time course by following the fluorescence emission of the fluorescein label at 520 nm (excitation 485 nm).

| Peptides used | LRQRIQCLRQKIAYL**RQQS**FDLKTQIAQLRQR |
|---|---|
| (CaMK Mol. Velcro) | EREICALEREIAYL**RQQS**FDLKTEIAQLERE |

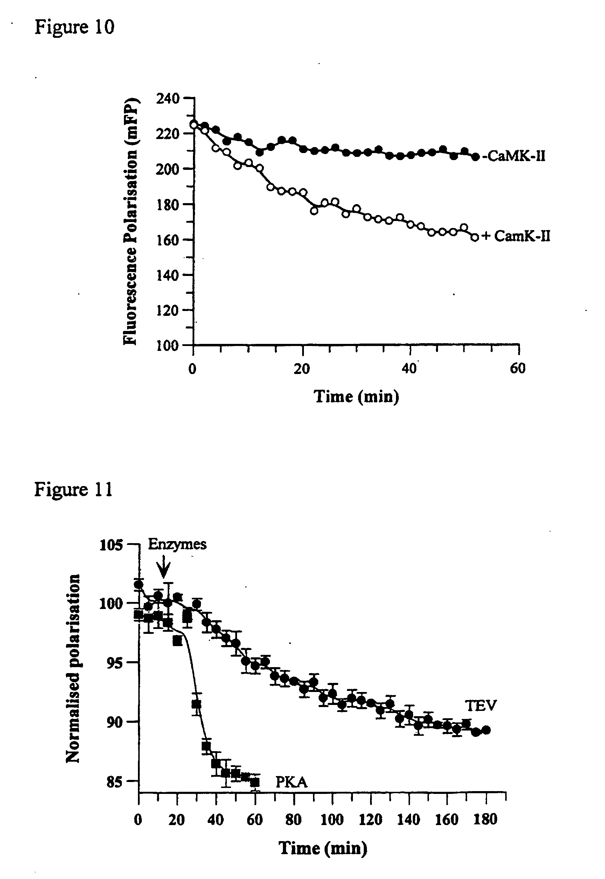

*(D) CaMK Fluorescence polarisation assay - Heterodimer (Figure 10)*

Coiled-coil reporter peptides containing phosphorylation sites for CaMK II were labelled with fluorescein (F) or biotin (B). Labelled peptides (5 µM total peptide, 4:1 ratio B:F label) were incubated at 30°C in 50 mM MOPS pH 7.2, 5 mM $MgCl_2$, 0.4 mM DTT, 0.1 mM $CaCl_2$, 0.1 mg/ml BSA, 0.5 µM calmodulin, and 1 mM ATP in a total volume of 100 µl in a microtitre plate. Streptavidin (0.16U) was added to each well and the samples were incubated at 30°C for 5min. Phosphorylation was initiated by the addition of CaMK (1 pmole), and monitored during the time course by measuring the fluorescence polarisation (excitation 485nm, emission 520nm).

| Peptides used | LRQRIQCLRQKIAYL**RQQS**FDLKTQIAQLRQR |
|---|---|
| (CaMK Mol. Velcro) | EREICALEREIAYL**RQQS**FDLKTEIAQLERE |

<u>Results</u>

**PKA Homodimer based kinase assay**

We have previously developed assays for protein kinase A activity using GCN4 homodimers peptide substrates comprising an introduced modification site for phosphorylation by the enzyme. These coiled coil peptide substrates have been used successfully to measure PK-A activity (see Figure 1) in a FRET configuration where one peptide partner is labelled with fluorescein (donor) and the partner peptide is labelled with rhodamine (acceptor).

**Heterodimer based kinase assay**

An improved version of the assay is based on the use of known natural or engineered heterodimers as peptide reporter substrates where one partner comprises a post translational modification site. The assay signal obtained with this configuration is significantly improved. For example, the addition of the acceptor peptide to the donor peptide in a 1:1 ratio results in about 80% FRET (one peptide is labelled with fluorescein and the partner is labelled with rhodamine) - see Figure 2. However, there was no observed collapse of the coiled coil partnership upon the addition of the modifying enzyme as reported by the lack of reversal of FRET

The introduction of a modification site on both peptides did result in reversal of the FRET signal. These findings are summarised diagrammatically in Figure 3.

Examples where this modification was used included phosphorylation sites for PKA and CaMK-II

(A) PKA Heterodimer FRET assays

Coiled-coil reporter peptides (Fos/jun based or Molecular Velcro based) each containing a phosphorylation site for PKA were labelled with fluorescein (F) or tetramethylrhodamine (R). Fluorescein-labelled peptide (donor 1.6 μM), was incubated at 30°C in 50 mM Histidine pH 7.0, 5 mM MgSO$_4$, 120 mM KCl, 5 mM NaF, 0.2 mg/ml BSA, and 1 mM ATP in a total volume of 100 μl in a microtitre plate. Once the signal has stabilised (ca. 2 min), the rhodamine-labelled peptide (acceptor, 1.6 μM) was added, this caused about 60% fluorescein quench due to FRET. Phosphorylation was then initiated by the addition of 3 pmoles of PKA), and the enzymic activity was monitored during the time course by following the recovery of fluorescence emission of fluorescein as the peptide pair dissociates as a function of modification (Figure 4).

(B) PKA FP assays

PKA assays using these heterodimer substrates were also reproduced in fluorescence polarisation mode.

In this type of assay only one peptide is labelled with fluorescein whereas the partner peptide is labelled with biotin. Since the fluorescence polarisation methodology is based on the detection of the rate of tumbling of macromolecules, whereby large molecules tumble very slowly giving a high polarisation value and small molecules tumble very fast giving a low polarisation value. The biotinylation of one peptide was carried out for the attachment of a streptavidin molecule, therefore increasing the size of the complex. The concept is based on the fact that once PKA phosphorylates the coiled coil, the dissociation of the partners yields a small peptide with a low polarisation value (Figure 5).

Coiled-coil reporter peptides containing phosphorylation sites for PKA were labelled with fluorescein (F) or biotin (B). Labelled peptides (3.2 μM total peptide, 1:1 ratio B:F label) were incubated at 30°C in 50 mM Histidine pH 7.0, 5 mM MgSO$_4$, 120 mM KCl, 5 mM NaF, 0.2 mg/ml BSA, and 1 mM ATP in a total volume of 100 μl in a microtitre plate. Streptavidin (0.1U) was added to each well and the samples were incubated at 30°C for 30 min. Once the signal had stabilised the phosphorylation was initiated by the addition of 3 pmoles), and the enzymic activity was monitored by following the decrease in fluorescence polarisation as indication of dissociation of the coiled coils.

Assay comparison using FRET and FP detection modes showed that both readouts are identical in terms of sensitivity (Figure 6 - volumes reduced to 20 μl (FRET) or 50μl (FP). Addition of PKA adjusted appropriately to give same concentration in well).

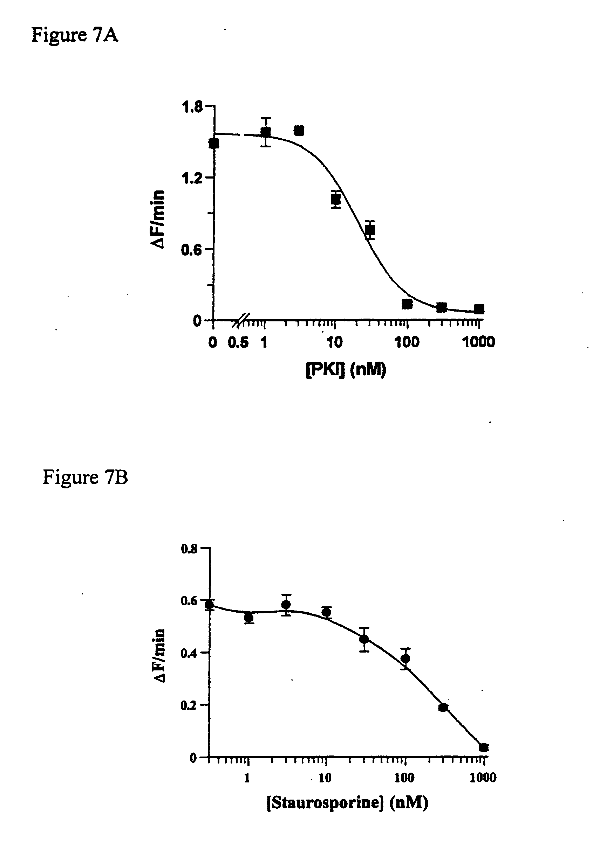

Kinetic parameters and inhibition profiles for PKA activity were also determined using specific peptide inhibitor DKA inhibitor amide 6-22 (calbiochem) as well as a general non-peptidic inhibitor (staurosporine) (Figure 7).

(C) CaMK-II FRET assays

The successful design of heterodimer coiled coil peptide substrates for PKA led to engineering sites for other kinases such as calmodulin-dependent protein kinase II and casein kinase I.

Under conditions similar to those described above for PKA, peptides containing phosphorylation sites for CaMK-II were labelled with fluorescein and rhodamine and when mixed together in a 1:1 ratio, they give about 70% FRET. When the enzyme is added the FRET is reversed indicating the dissociation of the coiled coil partnership (Figure 8).

Inhibition profiles were also obtained for the CamK-II using a specific peptide inhibitor Ca$^{2+}$/calmodulin kinase II inhibitor 201-301 (calbiochem) where kinetic parameters can be determined (Figure 9).

(D) CaMK-II Fluorescence Polarisation assays

These assays have been performed in a similar way to those described above for PKA whereby fluorescein labelled peptide was added to a biotinylated peptide partner attached to streptavidin. In the absence of the enzyme the complex exhibits high polarisation values, but upon the addition of the enzyme a decrease in polarisation was monitored indicating the dissociation of the coiled coils (Figure 10).

NB. All individual kinase assays have been shown to work at different temperatures (20-37°C).

**Reference Example 2 - Protease Assay**

*Fluorescence Polarisation TEV assay*

Coiled-coil reporter peptides containing cleavage sites for TEV were labelled with fluorescein (F) or biotin (B). Labelled peptides (10 μM total peptide, 6:1 ratio B:F label) were incubated for 30min at 30°C in 50 mM Tris pH 8.0, 0.5 mM EDTA, 1 mM DTT, and 0.2 mg/ml BSA in a total volume of 100 μl in a microtitre plate. Proteolytic cleavage was started by the addition of TEV (30U), and monitored during the time course by measuring the fluorescence polarisation (excitation 485 nm, emission 520 nm).

Peptides used      KGRMRCLEDRV**ENLYSQSY**HLENEVARLRRLVGELAAK

(TEV)      KGRMRCLEDRV**ENLYSQSY**HLENEVARLRRLVGELAAK

The results obtained showed that is possible to measure the activity of proteases using reporter polypeptides in fluorescence polarisation mode.

**Example 1 - Simultaneous assays**

<u>Materials and methods</u>

*PKA and TEV simultaneous assay (Homodimers) (figure 11)*

Coiled coil peptides containing PKA phosphorylation sites were labelled with coumarin or biotin. Coiled coil peptides containing TEV protease cleavage sites were labelled with fluorescein or biotin. The labelled peptides (4 μM PKA and 8 μM TEV, 6:1 ratio B:F each) were incubated at 30°C in 50mM Tris pH 7.8, 5 mM $MgSO_4$, 120 mM KCl, 5 mM NaF, 0.2mg/ml BSA, 0.14 units streptavidin and 1 mM ATP in a total volume of 100μl in a microtitre plate. Reactions were started by the addition of PKA (0.75 pmoles) and TEV (30units) to the wells. Enzyme activities was monitored during the time course by following fluorescence polarisation for coumarin (ex 390nm, em 450nm) and for fluorescein (ex 485nm, em 520nm).

Peptides used      KGRMRCLEDRV**ENLYSQSY**HLENEVARLRRLVGELAAK

(for TEV)      KGRMRCLEDRV**ENLYSQSY**HLENEVARLRRLVGELAAK

EREIKALEREI**RRLRRAS**QALEREIAQLERE

(PKA Mol. Velcro)      LRQRIQCLRYRI**RRLRRAS**QALRQRIAQLKQR

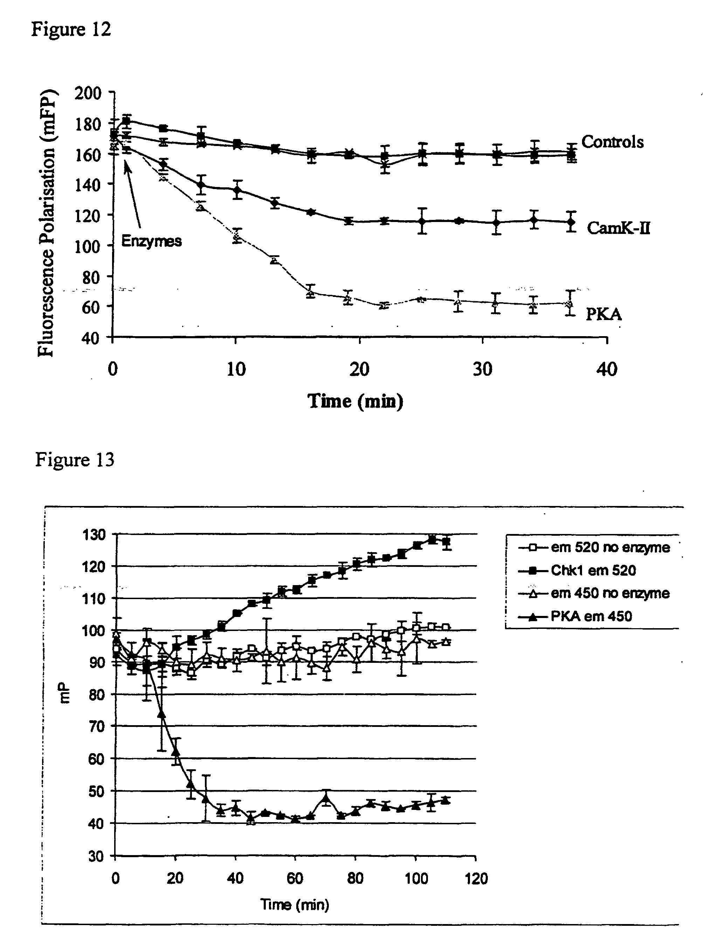

*PKA (Heterodimer) and CaMK (Heterodimer) (figure 12)*

Coiled coil peptides containing PKA phosphorylation sites were labelled with fluorescein or biotin. Coiled coil peptides containing CaMK II phosphorylation sites were labelled with coumarin or biotin. The labelled peptides (6 μM of each, 4:1 ratio B:C CaMK, 3:1 ratio B:F PKA) were incubated at 30°C in 50 mM MOPS pH 7.2, 10 mM MgSO$_4$, 0.4 mM DTT, 0.1 mM CaCl$_2$, 0.1 mg/ml BSA, 0.5 μM Calmodulin, 0.45 units streptavidin and 1 mM ATP in a total volume of 100 μl in a microtitre plate. Reactions were started by the addition of PKA (0.75pmoles) and CaMK (1 pmole) to the wells. Enzyme activity was monitored during the time course by following fluorescence polarisation for coumarin (ex 390nm, em 450nm) and for fluorescein (ex 485 nm, em 520 nm).

Peptides used      RMRCLEDEV**RRLRRKS**YHLANTVARLRQRVQELKAR

(PKA Fos/Jun)      LMCQLQDEV**RRLRRKS**YHLQNEVARLLREVQELEAE

                       LRQRIQCLRQKIAYL**RQQS**FDLKTQIAQLRQR

(CaMK Mol. Velcro)      EREICALEREIAYL**RQQS**FDLKTEIAQLERE

Results

The optimisation of two individual kinase assays described above in the reference example led us to examine if we can monitor the activities of at least two enzymes in a single sample.

The results shown in Figure 11 demonstrate that it is possible to monitor the activities of two different classes of enzymes (a kinase, PKA and a protease, TEV). These assays have been performed using FP detection mode.

In this assay TEV reporter peptides were labelled with biotin:streptavidin and fluorescein then mixed to form coiled coil partners in 100μl final volume. To this mixture, PKA reporter peptides which were labelled with biotin:streptavidin and coumarin were added.

To start the reactions, 30 units of TEV and 0.75pmoles of PKA were added sequentially to the mixture. The enzymic activities for TEV and PKA were measured simultaneously by following the decrease in polarisation at appropriate excitation and emission wavelengths (390, 450 nm for PKA and 485, 520 nm for TEV)

Using the same approach we have tested the possibility of monitoring the activities of two different kinases, PKA and CaMK-II in a single well. The results shown in Figure 12 demonstrate that this is indeed possible.

In this assay PKA reporter peptides were labelled with biotin:streptavidin and fluorescein then mixed to form coiled coil partners in 100μl final volume. To this mixture, CaMK reporter peptides labelled with biotin:streptavidin and coumarin were added. Reactions were started by addition of 0.75 pmoles of PKA and 1 pmole of CamK. The activities of PKA and CaMK were measured by following the decrease in polarisation at appropriate excitation and emission wavelengths (PKA: 485, 520nm, CaMKII 390, 450nm).

We have therefore also demonstrated that it is possible to measure the activity of two different kinases simultaneously.

**Example 2 - Simultaneous Assay of two serine threonine kinases, Chk1 and cAMP dependent protein kinase (PKA) by FP.**

Serine threonine kinases PKA and Chk1 can be assayed simultaneously using the natural binding partner reporters described below for Chk1 and a peptide based binding partner assay for PKA (WO99/11774).

**Methods**

Reagent sources

Chk1 enzyme and Ckhtide are from Upstate Biotechnology.

Chktide , Chk1 substrate peptide sequence:


## KKKVSRSGLYRSPS²¹⁶MPENLNRPR


Chktide labelling with fluorescein

Chktide is labelled by incubating for 2 hours at a concentration of 0.185mM in 100 mM NaHCO₃ pH 8.3, and 0.37 mM fluorescein-5EX (Molecular Probes) at room temperature. The labelled peptide is then dialysed against 3 changes of 50 mM Tris-HCl pH 7.4, 150 mM NaCl (200 ml) for a total of 18 hours.


Production of 14-3-3 ε

Under the control of the T7 promoter, the vector FS121 contains DNA encoding the 14-3-3ε (Genbank accession number U54778) protein in-frame to DNA encoding an amino terminal hexa-His tag. Fresh transformants of pFS121 in BRL(DE3) pLysS are used to inoculate 3 ml LB/ampicillin (100 μg/ml). The starter cultures is incubated overnight at 37°C with shaking. From these starter cultures 1ml is used to inoculate 400 ml Terrific Broth/ampicillin (100 μg/ml) in a 2L, baffled flask. Cultures are incubated at 37°C at 200 rpm for approximately 4 hr until the OD$_{600nm}$ has reached 0.5Abs units. At this point cultures are induced by adding IPTG to a concentration of 1mM and further incubated at 37°C for 4 hrs

Bacteria are harvested by centrifugation at 3000 rpm for 20 min. The bacterial pellet is resuspended in 25 ml lysis buffer (50 mM Phosphate pH 7.0, 300 mM NaCl, 2% Proteinase inhibitor cocktail (Sigma), 0.75mg/ml Lysozyme). Lysis of the resuspended cells is initiated by gentle stirring for 30 min at room temperature. Nonidet P-40 is added to a final concentration of 1% and lysis is continued for a further 20 min at room temperature. The partially lysed mixture is subjected to 3 cycles of freeze thawing in liquid nitrogen. Finally the cells are sonicated on ice using a 10 mm probe at high power. Sonication is performed on a pulse setting for a period of 4 min. The crude lysate is centrifuged at 15000 rpm for 30 min to remove cell debris. Hexa-His tagged proteins are purified from the cleared lysate using TALON® resin (Clontech). Proteins are bound to the resin in a batchwise manner by gentle shaking at room temperature for 30 min. Non-His-tagged proteins are removed by washing the resin at least twice with 10x bed volume of wash buffer (50 mM sodium phosphate pH 7.0, 300 mM NaCl, 5 mM fluorescence-blank imidazole ). The washed resin is loaded into a 2 ml column and the bound proteins are released with elution buffer (50 mM sodium phosphate pH 7.0, 300 mM NaCl, 150 mM florescence-blank imidazole). Elution is normally achieved within 5 ml. Purified proteins are stored at -80°C after snap freezing in liquid nitrogen in the presence of 10% glycerol.


PKA Peptide sequences:

## Peptide 1. ERE IKALERE IRRLRRA SQALERE IAQLERE


## Peptide 2. LRQR IQCLRYR IRRLRRA SQALRQR IAQLKQR


PKA peptides form coiled-coil dimers when they are non-phosphorylated. Each monomer has a PKA phosphorylation site. On phosphorylation by PKA, the dimers can no longer form. The association/disassociation can be measure using a fluorescence polarisation assay, where PKA peptide 1 is labelled with coumarin, and peptide 2 is labelled with biotin and bound to streptavidin. The tumbling rate of coumarin labelled peptide 1 is higher after PKA phosphorylation and dissociation from the dimer/streptavidin complex. At the same time, the activity of Chk1 is measured by the decrease in tumbling rate of phosphorylated fluorescein labelled Chktide substrate binding to 14-3-3ε protein.

The labelled PKA peptides (both at a concentration of 2.5 μM) are incubated at 30°C in a total volume of 50 μl (on a half area 96 well plate) in 20 mM MOPS pH 7.2, 10 mM MgCl₂ 25 mM β-glycerophosphate, 5 mM EGTA, 1mM sodium orthovanadate, 1 mM DTT, and 0.1 mM ATP, 3 μl 14-3-3 ε and 0.06U streptavidin. The samples are allowed to equilibrate for 5 min, the Chk1 (62 mU) and PKA (0.5 pmoles) are added (or an equal volume of H₂O for control samples). The fluorescence polarisation at 520 nm (excitation 485 nm) and at 450 nm (excitation 340 nm) is monitored over time as shown in Figure 13.

## EP 1 171 627 B1

**Claims**

1. A method of measuring simultaneously the activity of a first enzyme and a second enzyme in a system which method comprises

   (a) contacting a first binding domain and a first binding partner thereof with said first enzyme and contacting a second binding domain and a second binding partner thereof with said second enzyme; wherein

   (i) the first binding domain and/or binding partner comprise a site subject to post-translational modification by the first enzyme;

   (ii) modification of the site by the first enzyme affects the interaction between the first binding domain and first binding partner;

   (iii) the second binding domain and/or binding partner comprise a site subject to post-translational modification by the second enzyme; and

   (iv) modification of the site by the second enzyme affects the interaction between the second binding domain and second binding partner; and

   (b) simultaneously measuring the interaction between the first binding domain and the first binding partner and measuring the interaction between the second binding domain and the second binding partner.

2. A method according to claim 1 wherein:

   the first binding domain and first binding partner each comprise a detectable label such that when the first binding domain and first binding partner interact, a first detectable physical characteristic of one or both of the labels is altered; and

   the second binding domain and second binding partner each comprise a detectable label such that when the second binding domain and second binding partner interact, a second detectable physical characteristic of one or both of the labels is altered; and

   measurement of the interaction between the first binding domain and the first binding partner is performed by measuring changes in said first physical characteristic and measurement of the interaction between the second binding domain and the second binding partner is performed by measuring changes in said second physical characteristic, wherein the first physical characteristic is distinguishable from the second physical characteristic.

3. A method according to claim 2 wherein said physical characteristic is light emission/absorption.

4. A method according to claim 3, wherein said physical characteristic is fluorescent light emission.

5. A method according to claim 4 wherein one or more of the detectable labels is a fluorescent protein.

6. A method according to claim 4 or 5, wherein said method further comprises exciting said detectable label(s) and monitoring fluorescence emission.

7. A method according to any one of the preceding claims, wherein said first enzyme and/or said second enzyme is selected from a carbohydrate transferase, a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyl transferase, an NAD:Arginine ADP ribosyltransferase, a protease, a protein kinase and a protein phosphatase.

8. A method according to any one of the preceding claims wherein said method further comprises the step, prior to, during or after measurement of the interaction between the first binding domain and first binding partner and/or the interaction between the second binding domain and second binding partner of contacting said first enzyme and/or second enzyme with a compound which modulates the activity of said first and/or second enzyme.

9. A method of identifying a compound capable of modulating the activity of a first enzyme and/or second enzyme which method comprises

(i) contacting said first and/or second enzyme with a candidate substance

(ii) measuring the activity of the first enzyme and the second enzyme in said cells by the method of claim 1; and

(iii) determining whether the activity of the first and/or second enzyme is affected.

10. A set of two or more polypeptide pairs, each pair comprising a binding domain and a binding partner thereof comprising

(i) a site subject to post-translational modification by an enzyme wherein modification of the site by the enzyme affects the interaction between the binding domain and the binding partner; and

(ii) a detectable label on the binding domain and binding partner such that when the binding domain and the binding partner interact, a detectable physical characteristic of one or both of the labels is altered;

wherein for each pair, the enzyme which affects the interaction between the binding domain and the binding partner is different and the detectable physical characteristic can be distinguished from that of the other pairs.

11. Use of a set of polypeptide pairs according to claim 10 in a method for measuring simultaneously the activity of two or more enzymes.

**Patentansprüche**

1. Verfahren zum gleichzeitigen Messen der Aktivität eines ersten Enzyms und eines zweiten Enzyms in einem System, wobei das Verfahren folgendes umfaßt:

(a) in Kontaktbringen einer ersten Bindungsdomäne und eines ersten Bindungspartners davon mit dem ersten Enzym und in Kontaktbringen einer zweiten Bindungsdomäne und eines zweiten Bindungspartners davon mit dem zweiten Enzym, wobei

(i) die erste Bindungsdomäne und/oder der Bindungspartner eine Stelle umfassen, die einer posttranslationalen Modifikation durch das erste Enzym unterworfen wird,

(ii) eine Modifikation der Stelle durch das erste Enzym die Wechselwirkung zwischen der ersten Bindungsdomäne und dem ersten Bindungspartner beeinflußt,

(iii) die zweite Bindungsdomäne und/oder der Bindungspartner eine Stelle umfassen, die einer posttranslationaler Modifikation durch das zweite Enzym unterworfen wird, und

(iv) eine Modifikation der Stelle durch das zweite Enzym die Wechselwirkung zwischen der zweiten Bindungsdomäne und dem zweiten Bindungspartner beeinflußt und

(b) gleichzeitiges Messen der Wechselwirkung zwischen der ersten Bindungsdomäne und dem ersten Bindungspartner und Messen der Wechselwirkung zwischen der zweiten Bindungsdomäne und dem zweiten Bindungspartner.

2. Verfahren nach Anspruch 1, wobei
die erste Bindungsdomäne und der erste Bindungspartner jeweils eine feststellbare Markierung in der Weise umfassen, daß, wenn die erste Bindungsdomäne und der erste Bindungspartner miteinander wechselwirken, eine erste feststellbare physikalische Eigenschaft von einer oder beiden der Markierungen verändert wird, und
die zweite Bindungsdomäne und der zweite Bindungspartner jeweils eine feststellbare Markierung in der Weise umfassen, daß, wenn die zweite Bindungsdomäne und der zweite Bindungspartner miteinander wechselwirken, eine zweite feststellbare physikalische Eigenschaft von einer oder beiden der Markierungen verändert wird, und
eine Messung der Wechselwirkung zwischen der ersten Bindungsdomäne und dem ersten Bindungspartner durch Messen von Veränderungen in der ersten physikalischen Eigenschaft durchgeführt wird und ein Messen der Wechselwirkung zwischen der zweiten Bindungsdomäne und dem zweiten Bindungspartner durch Messen von Veränderungen in der zweiten physikalischen Eigenschaft durchgeführt wird, wobei die erste physikalische Eigenschaft von der zweiten physikalischen Eigenschaft unterscheidbar ist.

3. Verfahren nach Anspruch 2, wobei die physikalische Eigenschaft Lichtemission/-absorption ist.

4. Verfahren nach Anspruch 3, wobei die physikalische Eigenschaft Fluoreszenzlichtemission ist.

5. Verfahren nach Anspruch 4, wobei eine oder mehrere der feststellbaren Markierungen ein Fluoreszenzprotein ist.

6. Verfahren nach Anspruch 4 oder 5, wobei das Verfahren weiterhin umfaßt, daß man die feststellbare(n) Markierung(en) anregt und die Fluoreszenzemission überwacht.

7. Verfahren nach einem der vorangegangenen Ansprüche, wobei das erste Enzym und/oder das zweite Enzym unter einer Kohlenhydrattransferase, einem Ubiquitin-aktivierenden Enzym E1, einem Ubiquitin-konjugierenden Enzym E2, einem Ubiquitin-konjugierenden Enzym Ubc9, einer Ubiquitinproteinligase E3, einer Poly-(ADP-Ribose)-Polymerase, einer Fettsäuretransferase, einer NAD:Arginin-ADP-Ribosyltransferase, einer Protease, einer Proteinkinase und einer Proteinphosphatase ausgewählt ist.

8. Verfahren nach einem der vorangegangenen Ansprüche, wobei das Verfahren vor, während oder nach dem Messen der Wechselwirkung zwischen der ersten Bindungsdomäne und dem ersten Bindungspartner und/oder der Wechselwirkung zwischen der zweiten Bindungsdomäne und dem zweiten Bindungspartner weiterhin die Stufe umfaßt, bei der man das erste Enzym und/oder das zweite Enzym mit einer Verbindung in Kontakt bringt, welche die Aktivität des ersten und/oder zweiten Enzyms moduliert.

9. Verfahren zur Identifizierung einer Verbindung, die in der Lage ist, die Aktivität eines ersten Enzyms und/oder eines zweiten Enzyms zu modulieren, wobei das Verfahren folgendes umfaßt:

    (i) in Kontaktbringen des ersten und/oder zweiten Enzyms mit einer Kandidatensubstanz,

    (ii) Messen der Aktivität des ersten Enzyms und des zweiten Enzyms in den Zellen nach dem Verfahren von Anspruch 1 und

    (iii) Bestimmen, ob die Aktivität des ersten und/oder zweiten Enzyms beeinflußt wird.

10. Satz von zwei oder mehr Polypeptidpaaren, wobei jedes Paar eine Bindungsdomäne und einen Bindungspartner davon umfaßt, mit

    (i) einer Stelle, die posttranslationaler Modifikation durch ein Enzym unterworfen wird, wobei eine Modifikation der Stelle durch das Enzym die Wechselwirkung zwischen der Bindungsdomäne und dem Bindungspartner beeinflußt, und

    (ii) einer feststellbaren Markierung an der Bindungsdomäne und dem Bindungspartner in der Weise, daß, wenn die Bindungsdomäne und der Bindungspartner miteinander wechselwirken, eine feststellbare physikalische Eigenschaft von einer oder beiden der Markierungen verändert wird,

    wobei für jedes Paar das Enzym, welches die Wechselwirkung zwischen der Bindungsdomäne und dem Bindungspartner beeinflußt, verschieden ist und die feststellbare physikalische Eigenschaft von derjenigen des anderen Paares unterschieden werden kann.

11. Verwendung eines Satzes von Polypeptidpaaren nach Anspruch 10 in einem Verfahren zum gleichzeitigen Messen der Aktivität von zwei oder mehr Enzymen.

**Revendications**

1. Méthode pour mesurer simultanément l'activité d'une première enzyme et l'activité d'une seconde enzyme dans un système, méthode qui comprend les étapes consistant

(a) à mettre en contact un premier domaine de liaison et un premier partenaire de liaison de celui-ci avec ladite première enzyme et à mettre en contact un second domaine de liaison et un second partenaire de liaison de celui-ci avec ladite seconde enzyme ; où

(i) le premier domaine de liaison et/ou partenaire de liaison comprend un site sensible à la modification en post-traduction par la première enzyme ;
(ii) la modification du site par la première enzyme a un effet sur l'interaction entre le premier domaine de liaison et le premier partenaire de liaison ;
(iii) le second domaine de liaison et/ou partenaire de liaison comprend un site sensible à une modification en post-traduction par la seconde enzyme ; et
(iv) la modification du site par la seconde enzyme a un effet sur l'interaction entre le second domaine de liaison et le second partenaire de liaison ; et

(b) simultanément à mesurer l'interaction entre le premier domaine de liaison et le premier partenaire de liaison et à mesurer l'interaction entre le second domaine de liaison et le second partenaire de liaison.

2. Méthode suivant la revendication 1, dans laquelle:

le premier domaine de liaison et le premier partenaire de liaison comprennent chacun un marqueur détectable de telle sorte que, lorsque le premier domaine de liaison et le premier partenaire de liaison interagissent, une première caractéristique physique détectable de l'un des ou des deux marqueurs soit modifiée ; et
le second domaine de liaison et le second partenaire de liaison comprennent chacun un marqueur détectable de telle sorte que, lorsque le second domaine de liaison et le second partenaire de liaison interagissent, une seconde caractéristique physique détectable de l'un des ou des deux marqueurs soit modifiée ; et
la mesure de l'interaction entre le premier domaine de liaison et le premier partenaire de liaison est effectuée en mesurant les variations de ladite première caractéristique physique et la mesure de l'interaction entre le second domaine de liaison et le second partenaire de liaison est effectuée en mesurant les variations de ladite seconde caractéristique physique, la première caractéristique physique étant différenciable de la seconde caractéristique.

3. Méthode suivant la revendication 2, dans laquelle ladite caractéristique physique est une émission/absorption de lumière.

4. Méthode suivant la revendication 3, dans laquelle ladite caractéristique physique est une émission de lumière fluorescente.

5. Méthode suivant la revendication 4, dans laquelle un ou plusieurs des marqueurs détectables consistent en une protéine fluorescente.

6. Méthode suivant la revendication 4 ou 5, ladite méthode comprenant en outre l'excitation dudit ou desdits marqueurs détectables et le contrôle de l'émission de fluorescence.

7. Méthode suivant l'une quelconque des revendications précédentes, dans laquelle ladite première enzyme et/ou ladite seconde enzyme sont choisies entre une glucide-transférase, une enzyme d'activation d'ubiquitine E1, une enzyme de conjugaison d'ubiquitine E2, une enzyme de conjugaison d'ubiquitine Ubc9, une ubiquitine-protéine-ligase E3, une poly(ADP-ribose)polymérase, une acyle gras-transférase, une NAD:arginine-ADP-ribosyltransférase, une protéase, une protéine-kinase et une protéine-phosphatase.

8. Méthode suivant l'une quelconque des revendications précédentes, ladite méthode comprenant outre l'étape, avant, pendant ou après la mesure de l'interaction entre le premier domaine de liaison et le premier partenaire de liaison et/ou de l'interaction entre le second domaine de liaison et le second partenaire de liaison, de mise en contact de ladite première enzyme et/ou seconde enzyme avec un composé qui module l'activité de ladite première et/ou seconde enzyme.

9. Méthode pour identifier un composé capable de moduler l'activité d'une première enzyme et/ou d'une seconde enzyme, méthode qui comprend les étapes consistant

(i) à mettre en contact lesdites première et/ou seconde enzymes avec une substance candidate,

(ii) à mesurer l'activité de la première enzyme et de la seconde enzyme dans lesdites cellules par la méthode de la revendication 1 ; et

(iii) à déterminer si l'activité des première et/ou seconde enzymes est affectée.

10. Série de deux ou plus de deux paires de polypeptides, chaque paire comprenant un domaine de liaison et un partenaire de liaison de celui-ci, comprenant

(i) un site sensible de modification en post-traduction par une enzyme, la modification du site par l'enzyme ayant un effet sur l'interaction entre le domaine de liaison et le partenaire de liaison ; et

(ii) un marqueur détectable sur le domaine de liaison et le partenaire de liaison de telle sorte que, lorsque le domaine de liaison et le partenaire de liaison interagissent, une caractéristique physique détectable de l'un des ou des deux marqueurs soit modifiée,

où, pour chaque paire, l'enzyme qui a un effet sur l'interaction entre le domaine de liaison et le partenaire de liaison est différente et la caractéristique physique détectable peut être différenciée de celle des autres paires.

11. Utilisation d'une série de paires de polypeptides suivant la revendication 10 dans une méthode pour mesurer simultanément l'activité de deux ou plus de deux enzymes.

Figure 1

Figure 2

Figure 3

Good FRET          No reversal

Good FRET          Full reversal

Figure 4

Figure 5

Figure 6

Figure 7A

Figure 7B

Figure 8

Figure 9

Figure 10

Figure 11

Figure 12

Figure 13